(19) Europäisches Patentamt
European Patent Office
Office européen des brevets

(11) EP 0 544 874 B1

# (12) FASCICULE DE BREVET EUROPEEN

(45) Date de publication et mention de la délivrance du brevet: **08.04.1998 Bulletin 1998/15**

(21) Numéro de dépôt: **92912810.6**

(22) Date de dépôt: **23.06.1992**

(51) Int. Cl.$^6$: **A61K 9/127**, A61K 7/00, A61K 9/51

(86) Numéro de dépôt international: **PCT/FR92/00569**

(87) Numéro de publication internationale: **WO 93/00068 (07.01.1993 Gazette 1993/02)**

## (54) PROCEDE DE PREPARATION DE PARTICULES SUBMICRONIQUES EN PRESENCE DE VESICULES LIPIDIQUES ET COMPOSITIONS CORRESPONDANTES

HERSTELLUNGSVERFAHREN FÜR TEILCHEN IN SUBMIKRONGRÖSSE IN GEGENWART VON LIPIDVESIKELN SOWIE DIE ENTSPRECHENDEN ZUSAMMENSETZUNGEN

METHOD FOR PREPARING SUBMICRON PARTICLES IN THE PRESENCE OF LIPID VESICLES, AND CORRESPONDING COMPOSITIONS

(84) Etats contractants désignés:
**AT BE CH DE DK ES FR GB GR IT LI NL SE**

(30) Priorité: **24.06.1991 FR 9107727**

(43) Date de publication de la demande: **09.06.1993 Bulletin 1993/23**

(73) Titulaire: **L'OREAL
75008 Paris (FR)**

(72) Inventeurs:
- **MORANCAIS, Jean-Luc
F-77330 Ozoir-la-Ferrière (FR)**
- **LETY, Alain
F-77400 Lagny-sur-Marne (FR)**
- **VANLERBERGHE, Guy
F-77410 Villevaude (FR)**

(74) Mandataire: **Peuscet, Jacques
SCP Cabinet Peuscet et Autres,
78, avenue Raymond Poincaré
75116 Paris (FR)**

(56) Documents cités:
**EP-A- 0 344 040** **DD-A- 210 353**

- **WORLD PATENTS INDEX LATEST Derwent Publications Ltd., London, GB; AN 90-166618 (22) & JP,A,2 105 826 (CANON KK) 18 Avril 1990**
- **WORLD PATENTS INDEX LATEST Derwent Publications Ltd., London, GB; AN 90-166619 (22) & JP,A,2 105 827 (CANON KK) 18 Avril 1990**
- **J. AM. CHEM. SOC. vol. 106, 1984, WASHINGTON US pages 7359 - 7366; Y-M. TRICOT ET AL.: 'colloidal catalyst-coated semiconductors in surfactant vesicles: in situ generation of rh-coated cds particles in dihexadecylphosphate vesicles and their utilization for photosensitized charge separation and hydrogen generation'**
- **MAKROMOL. CHEM., RAPID COMMUN. vol. 8, 1987, BASEL CH pages 457 - 460; V.P. TORCHILIN ET AL.: 'polymerization of liposome-encapsulated hydrophilic monomers'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.1/3.4

## Description

La présente invention concerne un procédé de préparation de particules submicroniques de pigments ou de polymères en association avec des vésicules lipidiques à structure lamellaire dont les parois sont des feuillets constitués d'au moins une bi-couche lipidique, ainsi que les compositions obtenues par ce procédé.

De telles particules sont très recherchées notamment dans la préparation des pigments, des semi-conducteurs, des catalyseurs, des céramiques ou des émulsions photographiques. Dans le domaine des pigments, la diminution de la taille des particules permet principalement d'améliorer la stabilité des dispersions de pigments vis-à-vis du phénomène de sédimentation et d'augmenter le pouvoir couvrant desdits pigments.

Un des procédés utilisés à l'heure actuelle pour préparer des particules submicroniques consiste à encapsuler le précurseur de ces particules dans des vésicules lipidiques, puis à éliminer, généralement par filtration sur gel, l'excès de précurseur qui se trouve à l'extérieur desdites vésicules, puis à former ces particules in situ.

C'est ainsi que Mann S. et Hammington J.P. ont décrit, dans "Journal of Colloid and Interface Science", Vol 122, N°2, Avril 1988, pages 326-335, la préparation de particules métalliques d'oxyde de fer de dimensions comprises entre 5 et 12 nm. A cet effet, des solutions aqueuses de Fe(III) et/ou Fe(II) sont encapsulées dans des vésicules formées par des phosphatidylcholines ; les sels de fer extérieurs aux vésicules sont éliminés par chromatographie sur colonne échangeuse d'ions. Les pigments sont précipités à l'intérieur des vésicules par addition de soude ou d'ammoniaque. Les solutions de vésicules piégeant les précipités peuvent être stockées dans le noir à 4°C, pendant un laps de temps allant jusqu'à 8-10 jours.

Dans "Makromol Chem., Rapid Commun, 8, 457-460 (1987)", Torchili n V.P. et al ont décrit la préparation de particules polymériques de dimensions comprises entre 500 et 700 nm dans une dispersion de vésicules lipidiques ioniques dans une solution aqueuse de monomère. Les monomères extérieurs aux vésicules sont éliminés par chromatographie d'exclusion sur gel. Enfin, la polymérisation est effectuée in situ à l'intérieur des liposomes par irradiation ultraviolette.

Dans les demandes de brevets japonais publiées sous les n° 90 105 827 et 90 105 826, on a décrit la préparation de particules polymériques par dispersion de lipides dans une solution de monomères hydrosolubles. Le monomère se trouve encapsulé dans les vésicules ; dans le premier brevet, l'excès de monomère est éliminé par des procédés classiques, tels que la filtration sur gel ou la filtration sur résine échangeuse d'ions ; dans le second brevet, l'excès de monomère est rendu inactif par addition, à la dispersion de vésicules, d'un inhibiteur de polymérisation. La polymérisation est effectuée ensuite in situ par irradiation.

On a également décrit (Rafaeloff et al., J. Phys. Chem. 1985, 89 , 533-537) la préparation de particules submicroniques de sulfure de cadmium. Une solution aqueuse d'un complexe cadmium/EDTA (EDTA = Acide Ethylène-Diamino-Tétracétique) est encapsulée à l'intérieur de vésicules formées par du bromure (ou chlorure) de diméthyldioctadécylammonium. L'excès du complexe, c'est-à-dire celui qui se trouve à l'extérieur des vésicules, n'est pas éliminé. Cependant, les lipides formant les vésicules et les complexes sont choisis de telle sorte qu'il y ait sorption du complexe à la surface interne et externe des membranes vésiculaires. La précipitation du sulfure de cadmium est obtenue par bullage d'$H_2S$.

D'une façon analogue, Tricot Y.M. et Fendler J.M. (J. Phys. Chem., 1986, 90, 3369-3374) ont décrit un procédé suivant lequel on assure la sorption de cadmium à l'intérieur et à l'extérieur de la surface de membranes vésiculaires, en encapsulant du chlorure de cadmium dans des vésicules formées par du dihexadécylphosphate. Là également, étant donné la sorption des ions cadmium, il n'est pas nécessaire d'éliminer l'excès de solution aqueuse contenant ces ions à l'extérieur des vésicules. La précipitation du sulfure de cadmium est obtenue également par bullage d'$H_2S$.

On constate donc que les procédés de l'état antérieur de la technique, tels qu'ils viennent d'être décrits, exigent, ou bien que soit effectuée une séparation entre les vésicules et le milieu extérieur avant la formation des pigments ou des polymères ou bien qu'il y ait sorption du précurseur (de pigments ou de polymères) sur les vésicules.

La présente invention a pour objectif de simplifier les procédés de préparation de particules submicroniques de type pigments ou polymères, en association avec des vésicules lipidiques.

Il a maintenant été découvert, de façon surprenante, qu'il est possible, en vue de l'obtention de pigments ou polymères sous forme de particules submicroniques, de réaliser des réactions chimiques et, notamment, des polymérisations et des précipitations, dans un milieu microstructuré, lequel peut ne pas être constitué seulement par l'intérieur des vésicules, mais également par l'extérieur. Ceci permet de s'affranchir des exigences précitées, ce qui représente un avantage très important par rapport aux procédés antérieurs.

Selon la présente invention il est proposé un procédé mettant en jeu la solubilisation de précurseurs de polymères ou de pigments, dans le milieu interne et/ou dans le milieu de dispersion des vésicules, et l'addition des agents permettant la transformation des précurseurs en les pigments ou polymères attendus.

La présente invention a donc pour objet un procédé de préparation de particules submicroniques en présence de vésicules lipidiques, à partir d'au moins un précurseur desdites particules, à partir d'au moins un lipide et d'une première phase aqueuse dont une partie est destinée à être encapsulées dans les vésicules et, éventuellement une deuxième phase aqueuse destinée a faire partie du milieu de dispersion desdites vésicules, caractérisé par le fait qu'on

ajoute au moins un précurseur desdites particules dans la première phase aqueuse et/ou la deuxième phase aqueuse, avant et/ou pendant et/ou après la préparation de la phase vésiculaire, au moins un agent capable de transformer ledit (ou lesdits) précurseur(s) en les particules recherchées étant amené à agir sur celui-ci (ou ceux-ci) après son (ou leur) introduction dans le milieu de préparation, le précurseur contenu dans la phase aqueuse de dispersion n'étant pas éliminé avant l'introduction dudit agent de façon que la phase aqueuse de dispersion contienne des particules précipitées submicroniques.

Les précurseurs solubilisés dans l'eau peuvent être introduits à n'importe quelle étape de la préparation des dispersions vésiculaires. Il en résulte que les particules submicroniques pourront se trouver, soit essentiellement à l'intérieur des vésicules, soit essentiellement à l'extérieur des vésicules soit encore aussi bien à l'intérieur qu'à l'extérieur.

Il va de soi que le procédé faisant l'objet de l'invention s'applique à toute dispersion vésiculaire et quel que soit son mode de préparation.

On trouvera la description des divers modes de préparation dans "Les liposomes en biologie cellulaire et pharmacologie" Editions INSERM/John Libbey Eurotext, 1987, pages 6 à 18.

Conformément à un premier mode de réalisation de l'invention, on utilise au moins un précurseur susceptible de précipiter en particules submicroniques sous l'action d'au moins un agent de précipitation; conformément à un second mode de réalisation de l'invention, on utilise comme précurseur, au moins un monomère polymérisable, sur lequel on fait agir au moins un agent de polymérisation.

De préférence, on conduit la transformation du (ou des) précurseur(s) après la formation des vésicules.

On choisit le (ou les) lipide(s) parmi les lipides amphiphiles non-ioniques, les lipides amphiphiles ioniques, et les mélanges de lipides non-ionique(s) et ionique(s).

Parmi les lipides amphiphiles non-ioniques utilisables, on peut citer les lipides amphiphiles non-ioniques suivants :

(1) les dérivés de polyglycérol, linéaires ou ramifiés, de formule :

$$R^oO-\left[C_3H_5(OH)O\right]_{\bar{n}}-H \qquad (I)$$

formule dans laquelle :

. $-C_3H_5(OH)O$ est représenté par les structures suivantes prises en mélange ou séparément :

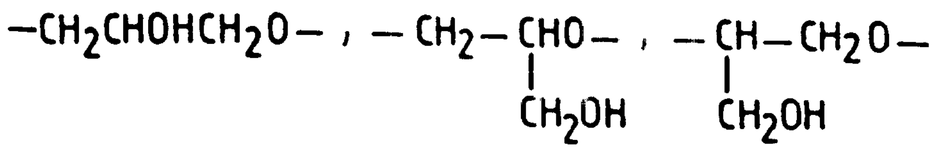

. $\bar{n}$ est une valeur statistique moyenne comprise entre 1 et 6 ;
. $R^o$ représente :

(a) une chaîne aliphatique, linéaire ou ramifiée, saturée ou insaturée, contenant de 12 à 30 atomes de carbone ; ou des radicaux hydrocarbonés des alcools de lanoline ; ou les restes d'alpha-diols à longue chaîne ;
(b) un reste $R^1CO$, où $R^1$ est un radical aliphatique, linéaire ou ramifié, en $C_{11}$-$C_{29}$ ;
(c) un reste $R^2$-[-$OC_2H_3(R^3)$-]- , où :

. $R^2$ peut prendre la signification (a) ou (b) donnée pour $R^o$ ;
. $OC_2H_3(R^3)$- est représenté par les structures suivantes, prises en mélange ou séparément :

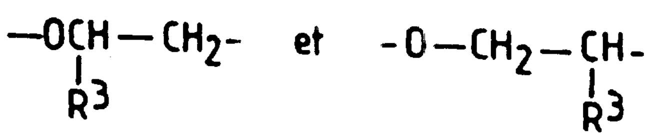

où $R^3$ prend la signification (a) donnée pour $R^o$ ;

(2) les éthers de polyglycérol, linéaires ou ramifiés, comportant deux chaînes grasses ;
(3) les alcools gras polyoxyéthylénés et les stérols et phytostérols polyoxyéthylénés ;
(4) les éthers de polyols ;
(5) les esters de polyols, oxyéthylénés ou non ;
(6) les glycolipides d'origine naturelle ou synthétique et notamment les éthers et les esters de glucose ;
(7) les hydroxyamides représentés par la formule (II) :

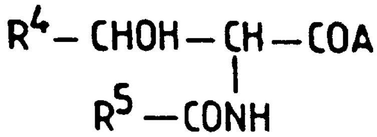

(II)

formule dans laquelle :

- $R^4$ désigne un radical alkyle ou alcényle en $C_7$-$C_{21}$ ;
- $R^5$ désigne un radical hydrocarboné, saturé ou insaturé en $C_7$-$C_{31}$ ;
- COA désigne un groupement choisi parmi les deux groupements suivants :

  . un reste

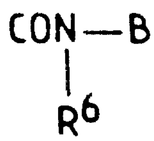

  où :

  . B est un radical alcoyle dérivé d'amines primaires ou secondaires, mono- ou polyhydroxylées ; et
  . $R^6$ désigne un atome d'hydrogène ou un radical méthyle, éthyle ou hydroxyéthyle ; et

  . un reste -COOZ, où Z représente le reste d'un polyol en $C_3$-$C_7$.

(8) les dérivés du glycérol décrits dans la demande de brevet PCT n°92/08685 et répondant à la formule :

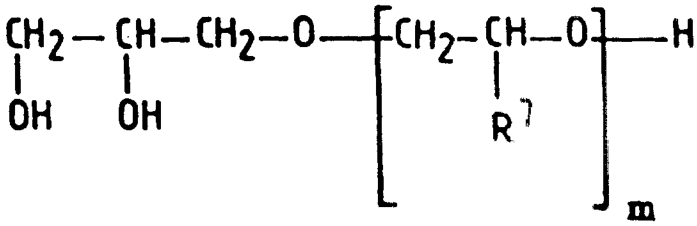

(III)

formule dans laquelle $R^7$ représente un radical alkyle linéaire en $C_{14}$ à $C_{18}$ ou un groupement $-CH_2A$ dans lequel A est $-OR_{14}$, $R_{14}$ représentant un radical alkyle linéaire en $C_{10}$-$C_{18}$ et, de préférence, en $C_{16}$, et m représente une valeur statistique moyenne supérieure à 1 et au plus égale à 3 et, en outre, lorsque $R_7 = -CH_2A$, m peut également représenter une valeur réelle (non statistique) égale à 2.
(9) les esters de glucose de formule :

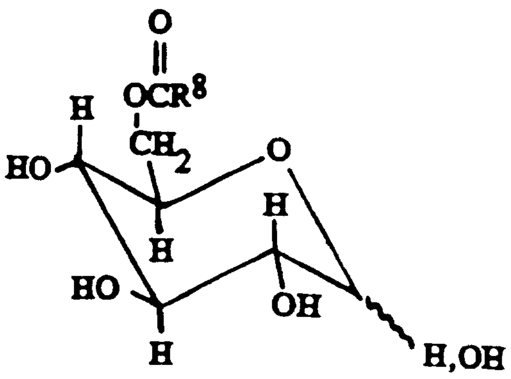

(IV)

formule dans laquelle $R^8$ représente une chaîne hydrocarbonée, linéaire, saturée ou insaturée, contenant de 9 à 17 atomes de carbone, ces esters étant décrits dans la demande de brevet européen n° 485 251.

Parmi les lipides amphiphiles ioniques utilisables, on peut citer :

- les phospholipides naturels, tels que la lécithine d'oeuf ou de soja et la sphingomyéline ;
- les phospholipides de synthèse, tels que la dipalmitoylphosphatidylcholine ou la lécithine hydrogénée ; et
- les composés anioniques, tels que les dialkylphosphates acides ou leurs sels comme, par exemple, le dicétylphosphate ;
- les composés cationiques dérivés ammonium quaternaire répondant à la formule (V) suivante :

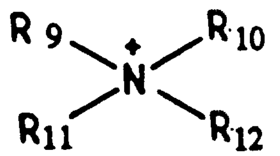

(V)

avec $R_9$ et $R_{10}$, identiques ou différents, représentant des radicaux alkyle en $C_{12}$-$C_{20}$ ; et $R_{11}$ et $R_{12}$, identiques ou différents, représentant des radicaux alkyle en $C_1$- $C_4$ ;
- les lipides polymérisables, comme ceux décrits par Ringsdorf et autres dans "Angewandte Chemie", vol. 27, n° 1, Janvier 1988, pages 129 et 137.

Par ailleurs, on peut associer aux lipides des additifs choisis parmi :

- les alcools et diols à longue chaîne ;
- les stérols, par exemple le cholestérol ;
- les amines à longue chaîne et leurs dérivés ammonium quaternaire ;
- les dihydroxyalkyl-amines ; les amines grasses polyoxyéthylénées ; les esters d'amino-alcools à longue chaîne ; et leurs sels et dérivés ammonium quaternaire ;
- les esters phosphoriques d'alcools gras ;
- les alkylsulfates, par exemple le cétylsulfate de sodium; et
- certains polymères, tels que les polypeptides et les protéines.

Egalement, on peut associer à la phase lipidique propre des vésicules, au moins un lipoprotide exempt de fonction sulfhydryle choisi parmi les dérivés mono- ou polyacylés d'aminoacides ou de polypeptides, dans lesquels le reste acyle $R_{13}$-CO comporte une chaîne hydrocarbonée $R_{13}$ en $C_{13}$-$C_{19}$, au moins une des fonctions qui relie la chaîne polypeptidique ou le reste d'aminoacide à la chaîne lipophile étant une fonction amide, les fonctions carboxyliques de la chaîne polypeptidique ou du reste aminoacide pouvant être partiellement ou totalement neutralisées par un ou plusieurs cations alcalins, un ion ammonium ou ammonium substitué dérivé d'une amine, ledit (ou lesdits lipoprotides) étant présent(s) à raison de 1 à 15% en poids par rapport au poids total de la phase lipidique propre, et/ou au moins un cholestérol sulfate de cation ammonium, alcalin ou alcalino-terreux, à raison de 1 à 50% en poids par rapport au poids de ladite phase lipidique propre.

Enfin, on peut associer à la phase lipidique de lipides non-ioniques au moins un cholestérol phosphate sous forme acide libre ou neutralisée par un cation ammonium, alcalin ou alcalino-terreux, à raison de 1 à 40% en poids par rapport au poids total de la phase lipidique propre.

Comme phase aqueuse E ou D, on utilise de l'eau, ou un mélange d'eau et d'au moins un solvant miscible à l'eau avantageusement choisi parmi les alcools en $C_1$-$C_7$ et les polyols d'alkyle en $C_1$-$C_5$.

Selon l'invention, on prépare avantageusement des particules submicroniques constituées par des pigments ou des polymères, en utilisant des agents de précipitation ou de polymérisation compatibles avec les vésicules sur les plans chimique et physicochimique et choisis notamment parmi les oxydants, les initiateurs de radicaux libres, les enzymes, les radiations, les acides et les bases, la précipitation des pigments pouvant résulter notamment d'une réaction d'hydrolyse en milieu organique, en milieu aqueux, d'une réaction à partir de chélates, d'une réaction de décomposition ou de double décomposition, d'une réaction redox, de la réaction chimique entre deux précurseurs organiques solubles dans le milieu réactionnel et conduisant à une espèce insoluble dans ce milieu, ou de l'action d'un mauvais solvant sur le précurseur de pigment considéré.

Ainsi, le procédé de l'invention s'applique à la préparation de pigments minéraux d'après les réactions suivantes :

- réactions d'hydrolyse en milieu organique (alcools) comme, par exemple :

$$\text{alcoxydes métalliques} \rightarrow TiO_2,\ BaTiO_3\ ,\ ZnO\ ;$$

réacations d'hydrolyse en milieu aqueux, comme, par exemple :

$$\text{sels métalliques} \rightarrow Cr(OH)_3,\ Al(OH)_3,\ TiO_2,\ Fe_2(SO_4)_3,\ Ag_2O;$$

réactions à partir de chélates, comme, par exemple:

$$\text{complexe } Mn^{+}/EDTA \xrightarrow{H_2S} MS$$

avec M = Pb, Cu, Zn et $1 \leq n \leq 4$

réactions de décomposition, comme, par exemple :

$$\text{Thiosulfate} \xrightarrow{H+} S$$

$$\text{Thioacétamide} \xrightarrow{M+/M++} MS \text{ avec M = Cd, Zn, Pb}$$

réactions redox, comme, par exemple :

$$\text{acide chloraurique} \xrightarrow{CH_2O} Au$$

$$\text{acide sélénieux} \xrightarrow{\text{hydrazine}} Se$$

$$BrO_3^{-} \xrightarrow[\text{en présence de Ag}]{HNO_2} AgBr$$

$$\text{Tartrate cuivrique} \xrightarrow{\text{glucose}} Cu_2O$$

$$Fe(CO)_5 \xrightarrow[\text{dans EtOH}]{H_2O_2} Fe(OH)_3$$

avec Et = éthyle
réactions de double décomposition, comme, par exemple :

$$NH_4Cl + AgNO_3 \rightarrow AgCl + NH_4NO_3$$

$$KIO_3 + Pb(NO_3)_2 \rightarrow Pb(IO_3)_2$$

$$KIO_3 + La(NO_3)_3 \rightarrow La(IO)_3)_3$$

Perchlorate Ferrique + Acide phosphorique $\rightarrow FePO_4$

précipitation par un mauvais solvant.

Dans un mode de réalisation préféré, on prépare des particules submicroniques de pigments obtenus par oxydation ou par polymérisation oxydante ou enzymatique d'au moins un composé indolique et/ou d'au moins d'un composé indolinique.

On choisit le composé indolique, notamment, parmi ceux répondant à la formule :

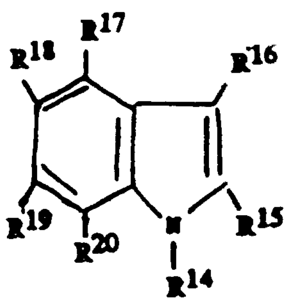

(VI)

formule dans laquelle :

- $R^{14}$ et $R^{16}$ représentent, indépendamment l'un de l'autre, l'hydrogène ou un radical alkyle en $C_1$-$C_4$ ;
- $R^{15}$ représente l'hydrogène, ou un radical alkyle en $C_1$-$C_4$, un groupe carboxyle ou (alcoxy en $C_1$-$C_4$)-carbonyle ;
- $R^{17}$ et $R^{20}$ représentent, indépendamment l'un de l'autre, l'hydrogène, un groupe hydroxy, un groupe alkyle en $C_1$-$C_4$, un groupe amino, alcoxy en $C_1$-$C_4$, (alcoxy en $C_1$-$C_4$)-oxy, (acyl en $C_2$-$C_4$)-amino ;
- $R^{18}$ représente l'hydrogène, un groupe hydroxy, alcoxy en $C_1$-$C_4$, un radical alkyle en $C_1$-$C_4$, un groupe halogène, amino, (acyl en $C_2$-$C_4$)-oxy, (acyl en $C_2$-$C_4$)-amino, triméthylsilyloxy ;
- $R^{19}$ représente l'hydrogène, un groupe hydroxy, alcoxy en $C_1$-$C_4$, amino, (acyl en $C_2$-$C_4$)-oxy (acyl en $C_2$-$C_4$)-amino, triméthylsilyloxy, (hydroxyalkyl en $C_2$-$C_4$)-amino ;
- $R^{18}$ et $R^{19}$ pouvant également former, conjointement avec les atomes de carbone auxquels ils sont rattachés, un cycle méthylènedioxy éventuellement substitué par un groupement alkyle en $C_1$-$C_4$ ou alcoxy en $C_1$-$C_4$, ou bien un cycle carbonyldioxy ;

  avec la condition qu'au moins l'un des radicaux $R^{17}$ à $R^{20}$ représente un groupement OZ ou $NHR^{21}$ avec au plus un des radicaux $R^{17}$ à $R^{20}$ représentant $NHR^{21}$ ; et au plus deux des radicaux $R^{17}$ à $R^{20}$ représentent OZ et, dans le cas où Z représente l'hydrogène, les deux OH sont dans les positions 5 et 6 ; et au moins l'un des radicaux $R^{17}$ à $R^{20}$ représente l'hydrogène, et dans le cas où un seul de ces radicaux représente l'hydrogène, un seul radical parmi $R^{17}$ à $R^{20}$ représente alors $NHR^{21}$ ou OZ, les autres radicaux représentant un radical alkyle en $C_1$-$C_4$ ; $R^{21}$ désignant l'hydrogène, un groupe acyle en $C_2$-$C_4$, hydroxyalkyle en $C_2$-$C_4$, et Z désignant l'hydrogène, un groupe acyle en $C_2$-$C_{14}$ alkyle en $C_1$-$C_4$, ou triméthylsilyle ;

et leurs sels de métaux alcalins, alcalino-terreux, d'ammonium ou d'amines, ainsi que les chlorhydrates, les bromhydrates, les sulfates et les méthanesulfonates correspondants.

Ces composés indoliques sont choisis, de préférence, dans le groupe formé par le 4-hydroxyindole, le 5-hydroxyindole, le 6-hydroxyindole, le 7-hydroxyindole, le 4-hydroxy 5-méthoxyindole, le 4-hydroxy 5-éthoxyindole , le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-hydroxyindole, le 3-méthyl 5,6-hydroxyindole, le 2,3-diméthyl 5,6-dihydroxyindole, le 2-carboxyl 5,6-dihydroxyindole, le 4-hydroxy 5-méthyl indole, le 2-carboxyl 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxyl 5-méthyl-indole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6 β-hydroxyéthylaminoindole, le 6-amino 2,3-diméthylindole, le 6-amino 2,3,4,5-tétraméthylindole, le 6-amino 2,3,5-triméthylindole, le 6-amino 2,3,5-triméthylindole, le 6-amino 2,3,6-triméthylindole, le 5,6-diacétoxyindole, le 5-méthoxy 6-acétoxyindole et le 5,6-diméthylindole, et les sels d'addition de ces composés. Le 5,6-dihydroxyindole et le 6-hydroxyindole sont particulièrement préférés.

On choisit le composé indolinique notamment parmi ceux répondant à la formule :

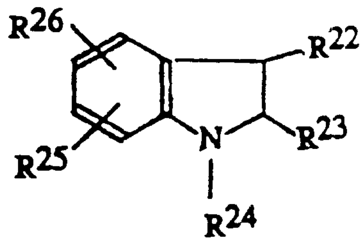

(VII)

formule dans laquelle :

- $R^{22}$ et $R^{24}$ représentent, indépendamment l'un de l'autre, un atome d'hydrogène ou un radical alkyle en $C_1$-$C_4$ ;
- $R^{23}$ représente un atome d'hydrogène, un radical alkyle en $C_1$-$C_4$, -------------un groupe carboxyle ou alcoxy ($C_1$-$C_4$) carbonyle ;
- $R^{25}$ désigne un atome d'hydrogène, un radical alkyle en $C_1$-$C_4$, hydroxyle, alcoxy ($C_1$-$C_4$), amino ou alkylamino en $C_1$-$C_{10}$ ou halogène ;
- $R^{26}$ désigne un atome d'hydrogène, un groupe hydroxyle, alcoxy en $C_1$-$C_4$ ou amino ; avec la condition qu'au moins un des radicaux $R^{25}$ ou $R^{26}$ désigne un groupe hydroxyle, alcoxy ou amino ; et sous réserve que, lorsque $R^{26}$ désigne un groupe amino, $R^{25}$ ne peut désigner un radical alkylamino ;
- $R^{25}$ et $R^{26}$ pouvant également former un cycle alkylènedioxy en $C_1$-$C_2$, lorsqu'ils sont en position 5 et 6 ;

ainsi que parmi les sels correspondants.

Parmi les composés répondant à la formule (VII), les composés préférés utilisés conformément à l'invention, sont choisis dans le groupe formé par la 5,6-dihydroxyindoline, la 6-hydroxyindoline, la 5,6-méthylènedioxyindoline, la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6-aminoindoline, la 5,6-diaminoindoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amino 6-chloroindoline, la 4-amino 6-iodoindoline, la 4-amino 5-bromoindoline, la 4-amino 5-hydroxyindoline, la 4-amino 7-hydroxyindoline, la 4-amino 5-méthoxyindoline, la 4-amino 7-méthoxyindoline, la 5-aminoindoline, la 2,3-diméthyl 5-aminoindoline, la 1-méthyl 5-aminoindoline, la 2-méthyl 5-aminoindoline, la 5-N-(1-méthylhexyl)aminoindoline, la 5,6-diméthoxyindoline et la 5,6-dihydroxy 2-carboxyindoline.

Dans les composés de formule (VII), les radicaux alkyle en $C_1$-$C_4$ désignent de préférence méthyle, éthyle propyle, isopropyle, butyle, isobutyle : les radicaux alkyle en $C_1$-$C_{10}$ désignent, de préférence, méthyle, éthyle, propyle, isopropyle, butyle, isobutyle, 1-méthylhexyle, 1-méthylheptyle, 1-méthyloctyle ; les radicaux alcoxy désignent, de préférence, méthoxy, éthoxy, propoxy et butoxy ; halogène désigne, de préférence, brome, chlore ou iode.

Les sels des composés de formule (VII) sont, en particulier, des chlorhydrates, bromhydrates, sulfates, méthanesulfonates ou des sels de métaux alcalins, alcalino-terreux, d'ammonium ou d'amines. Les bromhydrates des composés de formule (VII) sont particulièrement préférés.

L'oxydation du composé indolique de formule (VI) ou du composé indolinique de formule (VII) peut s'effectuer en milieu aqueux ou eau-solvant(s), à l'air, en présence ou non d'un agent alcalin et/ou d'un catalyseur métallique d'oxydation. Un catalyseur métallique d'oxydation préféré est constitué par l'ion cuivrique.

Le milieu réactionnel est, de préférence, constitué par de l'eau et peut, le cas échéant, être constitué par un mélange d'eau et d'au moins un solvant choisi de telle façon qu'il solubilisera rapidement le composé indolique de formule (VI) ou le composé indolinique de formule (VII). Parmi ces solvants, on peut citer, à titre d'exemples, les alcools inférieurs en $C_1$-$C_7$, tels que l'alcool éthylique, l'alcool propylique ou isopropylique, l'alcool tert-butylique, les alkylèneglycols, tels que l'éthylèneglycol, le propylèneglycol, les alkyléthers d'alkylèneglycols, tels que les éthers mono-méthylique, monoéthylique et monobutylique de l'éthylèneglycol, les monométhyléthers du propylèneglycol et du dipropylèneglycol, et le lactate de méthyle.

L'oxydation peut également s'effectuer en mettant en oeuvre le peroxyde d'hydrogène en présence d'un agent alcalin, tel que, de préférence, l'ammoniaque, ou en présence d'un ion iodure, l'iodure étant, de préférence, l'iodure d'un métal alcalin, alcalinoterreux ou d'ammonium.

On peut également procéder à l'oxydation en utilisant l'acide periodique et ses sels hydrosolubles et dérivés, les permanganates et bichromates, tels que de sodium ou de potassium, l'hypochlorite de sodium, le ferricyanure de potassium, le persulfate d'ammonium, l'oxyde d'argent, l'oxyde de plomb, le chlorure ferrique, le nitrite de sodium, les sels de terres rares dont, notamment, le cérium, et des oxydants orgamques choisis parmi les ortho- et parabenzoquinones, les ortho- et parabenzoquinones mono- ou diimines, les 1,2- et 1,4-naphtoquinones, les 1,2- et 1,4-naphtoquinones mono- ou diimines. Le sel d'acide periodique préféré est le periodate de sodium.

Il est possible d'activer les agents oxydants par un modificateur de pH. Par exemple, lors de l'utilisation du système iodure/peroxyde d'hydrogène, on met en oeuvre, de préférence, un milieu alcalin, ce qui permet d'activer la réaction.

On peut également envisager une oxydation enzymatique.

Les pigments selon l'invention peuvent également provenir de la polymérisation oxydante ou enzymatique de précurseurs tels que la L-tyrosine, la L-dopa, le catéchol et leurs dérivés.

Selon l'invention, on peut également préparer des particules de polymères d'acrylamide et de bisacrylamide.

On peut également préparer des particules de polymères par précipitation du polymère en solution sous l'effet d'un changement de pH.

Conformément à un mode particulier de réalisation de la présente invention, on prépare une phase lamellaire, en solubilisant le (ou les) lipide(s) devant former la paroi des vésicules dans un solvant, on fait évaporer le solvant sous une pression réduite, puis on mélange l'association lipidique ainsi formée avec la phase aqueuse E, on homogénéise, on chauffe à une température de 10-150°C, de préférence 40-80°C, pendant 0,25-2 heures et on fait revenir à la température ambiante, en réitérant au moins une fois le cycle : homogénéisation - chauffage - retour à la température ambiante ; on utilise avantageusement, comme solvant, le dichlorométhane, le chloroforme, l'acétate d'éthyle, l'acétate de butyle, le formiate d'éthyle, l'hexane, le cyclohexane, le toluène, l'éther de pétrole, le méthanol, l'éthanol, le propanol, l'éther méthylique, l'éther éthylique et les mélanges d'au moins deux d'entre eux.

Conformément à un mode particulier de mise en oeuvre de la présente invention, on prépare avantageusement une phase lamellaire dans laquelle la concentration en lipide(s) se situe entre 10 et 90 % en poids, de préférence entre 20 et 80 % en poids ; on prépare la phase vésiculaire en homogénéisant le mélange de la phase lamellaire et de la phase D par au moins un moyen mécanique de type secouage et/ou ultrasons : on prépare la phase vésiculaire à une température comprise entre 10 et 150°C, de préférence entre 40 et 80°C ; on prépare une phase vésiculaire, dans laquelle la concentration en lipides se situe entre 1 et 90 % en poids, de préférence, entre 5 et 20 % en poids ; les vésicules ont des dimensions comprises entre 20 et 3 000 nm, de préférence, comprises entre 20 et 500 nm.

On obtient avantageusement un milieu dans lequel sont dispersées des vésicules lipidiques et des particules submicroniques, lesdites particules étant contenues dans lesdites vésicules et/ou indépendantes de celles-ci, le volume desdites vésicules et, le cas échéant, des particules indépendantes de celle-ci, représentant 5 à 90 %, de préférence, 10 à 70 % du volume du milieu.

L'invention a aussi pour objet une composition obtenue par le procédé ci-dessus défini.

Selon un mode de réalisation particulier, la composition selon l'invention comporte en milieu aqueux de 0,1 à 20 % et, de préférence, de 0,1 à 5 % en poids des particules de pigments ayant des dimensions comprises entre 20 et 500 nm, les pourcentages étant donnés par rapport au poids total de la composition.

Pour mieux faire comprendre l'objet de l'invention, on va en décrire maintenant, à titre d'exemples purement illustratifs et non limitatifs, plusieurs modes de mise en oeuvre. Dans ces exemples, les pourcentages indiqués sont donnés en poids, sauf indication contraire.

**EXEMPLE 1 :**

*Première étape* :

On utilise une phase lipidique formulée comme suit :

- Lipide amphiphile non-ionique de formule (VIII) :

$$C_{16}H_{33}O-\left[C_3H_5(OH)O\right]_{\bar{n}}-H \qquad \text{(VIII)}$$

formule dans laquelle :

. $-C_3H_5(OH)O-$ est représenté par les structures suivantes, prises en mélange ou séparément :

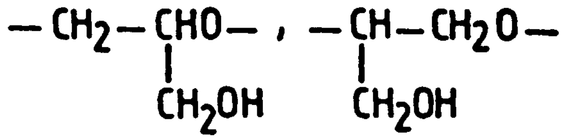

. et $\bar{n}$ est une valeur statistique moyenne égale à 3 ........................................................... 1,43 g

- Cholestérol ........................................................... 1,43 g
- Dihexadécylphosphate de sodium .................................. 0,14 g

On introduit les constituants précités dans un ballon de 50 ml, puis on les dissout dans 10 ml de dichlorométhane. Ensuite, le solvant est évaporé à 40°C sous pression réduite, par paliers successifs, depuis la pression ambiante jusqu'à environ 500 Pa, à l'aide d'un évaporateur rotatif.

*Deuxième étape :*

On prélève le film lipidique formé à l'issue de la première étape, et on le place dans un flacon de 60 ml. On ajoute aux lipides 3,00 g d'eau. On soumet le mélange obtenu au cycle suivant, lequel est répété 4 fois : homogénéisation à l'aide d'une spatule - chauffage en étuve à 75°C pendant 1 heure - retour à la température ambiante par refroidissement spontané à l'air.

*Troisième étape :*

A la phase lamellaire issue de la deuxième étape, on ajoute 22,40 g d'une solution contenant 1,21 % de 5,6-dihydroxyindole et 0,5 1 % d'iodure de potassium dans l'eau. Le mélange ainsi obtenu est agité par secouage, pendant 15 minutes, à la température ambiante.

*Quatrième étape :*

A la dispersion obtenue à l'issue de la troisième étape, on ajoute, sous agitation douce, 0,50 g d'une solution d'hydroxyde de sodium 0,1 M dans l'eau. LA pH de la dispersion est égal à 7,47.

*Cinquième étape :*

A la dispersion obtenue à l'issue de la quatrième étape, on ajoute 1,40 g d'une solution de peroxyde d'hydrogène à 22,4 volumes dans l'eau. Le mélange, porté à la température de 30°C, est traité pendant 8 minutes à l'aide d'un homogénéisateur à ultrasons. La dispersion de vésicules obtenue est ensuite laissée au repos à la température

ambiante.

La composition pondérale de la préparation ainsi réalisée est la suivante :

| | |
|---|---|
| - Lipide non-ionique de formule (VIII) | 4,73 % |
| - Cholestérol | 4,73 % |
| - Dihexadécylphosphate de sodium | 0,46 % |
| - 5,6-dihydroxyindole polymérisé | 0,90 % |
| - Excipient aqueux qsp | 100 % |

La fraction volumique des particules dispersées dans cette préparation (vésicules et pigments à base de 5,6-dihydroxyindole polymérisé) est égale à 38 %, après un jour de conservation à la température ambiante.

La dimension moyenne des particules dispersées (vésicules et pigments), mesurée après 7 jours de conservation à température ambiante, est égale à 281 ± 3 nm ; et la dimension moyenne des pigments, mesurée après 14 jours de conservation à température ambiante, est égale à 202 ± 7 nm.

**<u>EXEMPLE 2</u> :**

*Première étape :*

On utilise une phase lipidique formulée comme suit :

| | |
|---|---|
| - Lipide non-ionique de formule (VIII) | 0,71 g |
| - Cholestérol | 0,71 g |
| - Dihexadécylphosphate de sodium | 0,08 g |

On procède ensuite comme pour la première étape de l'exemple 1.

*Deuxième étape :*

On procède comme pour la deuxième étape de l'exemple 1, excepté que l'on ajoute 1,50 g d'eau.

*Troisième étape :*

A la phase lamellaire issue de la deuxième étape, on ajoute 25,30 g d'une solution contenant 1,13 % de 5,6-dihydroxyindole et 0,48 % d'iodure de potassium dans l'eau. Le mélange ainsi obtenu est agité par secouage pendant 15 minutes à' la température ambiante.

*Quatrième étape :*

A la dispersion obtenue à l'issue de la troisième étape, on ajoute, sous agitation douce, 0,70 g d'une solution d'hydroxyde de sodium 0,1 M dans l'eau. Le pH de la dispersion est égal à 7,50.

*Cinquième étape :*

On procède comme pour la cinquième étape de l'exemple 1, excepté que l'on utilise 1,50 g de solution de peroxyde d'hydrogène à 22,4 volumes dans l'eau.

La composition pondérale de la préparation ainsi réalisée est la suivante :

| | |
|---|---|
| - Lipide non-ionique de formule (VIII) | 2,32 % |
| - Cholestérol | 2,32 % |
| - Dihexadécylphosphate de sodium | 0,26 % |
| - 5,6-dihydroxyindole polymérisé | 0,94 % |
| - Excipient aqueux qsp | 100 % |

La fraction volumique des particules dispersées dans cette préparation (vésicules et pigments à base de 5,6-dihydroxyindole polymérisé) est égale à 29 % après un jour de conservation à la température ambiante.

La dimension moyenne des particules dispersées (vésicules et pigments), mesurée après un jour de conservation à température ambiante, est égale à 326 ± 8 nm ; et la dimension moyenne des pigments, mesurée après un jour de conservation à température ambiante, est égale à 188 ± 3 nm.

**<u>EXEMPLE 3</u> :**

*Première étape :*

On utilise une phase lipidique formulée comme suit :

| | |
|---|---|
| - Lipide non-ionique de formule (VIII) | 1,16 g |
| - Cholestérol | 1,16 g |
| - Dihexadécylphosphate de sodium | 0,12 g |

On procède comme pour la première étape de l'exemple 1.

*Deuxième étape :*

On procède comme pour la deuxième étape dans l'exemple 1, excepté que l'on utilise 2,45 g d'eau

*Troisième étape :*

A la phase lamellaire issue de la deuxième étape, on ajoute 19,21 g d'une solution contenant 1,00 % de 5,6-dihydroxyindole dans l'eau. Le mélange ainsi obtenu est agité par secouage pendant 15 minutes à la température ambiante

*Quatrième étape :*

A la dispersion issue de la troisième étape, on ajoute 0,54 g d'une solution de peroxyde d'hydrogène à 17,5 volumes dans l'eau. Le mélange ainsi obtenu est traité pendant 8 minutes à l'aide d'un homogénéisateur à ultrasons.

*Cinquième étape :*

La dispersion de vésicules issue de la quatrième étape est maintenue sous agitation mécanique douce et portée à une température de 80°C pendant 4 heures.

La composition pondérale de la préparation ainsi réalisée est la suivante :

| | |
|---|---|
| - Lipide non-ionique de formule (VIII) | 4,64 % |
| - Cholestérol | 4,64 % |
| - Dihexadécylphosphate de sodium | 0,49 % |
| - 5,6-dihydroxyindole polymérisé | 0,78 % |
| - Excipient aqueux qsp | 100 % |

La fraction volumique des particules dispersées dans cette préparation (vésicules et pigments à base de 5,6-dihydroxyindole polymérisé) est égale à 49 % après un jour de conservation à la température ambiante.

La dimension moyenne des particules dispersées (vésicules et pigments), mesurée après un jour de conservation à température ambiante, est égale à 232 ± 6 nm ; et la dimension moyenne des pigments, mesurée après un jour de conservation à température ambiante, est égale à 183 ± 2 nm.

**EXEMPLE 4 :**

*Première étape :*

On utilise une phase lipidique formulée comme suit:

| | |
|---|---|
| - Lipide non-ionique de formule (VIII) | 1,25 g |
| - 2-doicosanoylamino-1,3-octadécanediol | 0,26 g |
| - Cholestérol | 0,99 g |
| - Dihexadécylphosphate de sodium | 0,13 g |

On procède comme pour la première étape de l'exemple 1.

*Deuxième étape :*

On procède comme pour la deuxième étape de l'exemple 1, excepté que l'on utilise 2,64 g d'eau.

*Troisième étape :*

On procède comme pour la troisième étape de l'exemple 1, excepté que l'on ajoute 19,30 g d'une solution contenant 1,00 % de 5,6-dihydroxyindole et 0,42 % d'iodure de potassium dans l'eau.

*Quatrième étape :*

A la dispersion obtenue à l'issue de la troisième étape, on ajoute, sous agitation douce, 0,20 g d'une solution d'hydroxyde de sodium 0,1 M d'eau. Le pH de la dispersion est égal à 7,58.

*Cinquième étape :*

On procède comme pour la cinquième étape de l'exemple 1.

La composition pondérale de la préparation ainsi réalisée est la suivante :

| | |
|---|---|
| - Lipide non-ionique de formule (VIII) | 4,83 % |
| - 2-doicosanoylamino-1,3-octadécanediol | 1,02 % |
| - Cholestérol | 3,81 % |
| - Dihexadécylphosphate de sodium | 0,51 % |
| - 5,6-dihydroxyindole polymérisé | 0,75 % |
| - Excipient aqueux qsp | 100 % |

La fraction volumique des particules dispersées dans cette préparation (vésicules et pigments à base de 5,6-dihydroxyindole polymérisé) est égale à 35 % après 12 jours de conservation à température ambiante.

La dimension moyenne des particules dispersées (vésicules et pigments), mesurée après 12 jours de conservation à température ambiante, est égale à 243 ± 4 nm ; et la dimension moyenne des pigments, mesurée après 21 jours de conservation à température ambiante, est égale à 192 ± 3 nm.

**EXEMPLE 5 :**

*Première étape :*

On utilise une phase lipidique formulée comme suit :

- Lipide amphiphile non-ionique de formule :

$$C_{12}H_{25}\text{-}[\text{-}OC_2H_3(R')\text{-}]\text{-}O\text{-}[\text{-}C_3H_5(OH)O\text{-}]_{\bar{n}}\text{-}H \qquad (IX)$$

formule dans laquelle :

. $-C_3H_5(OH)O-$ est constitué par un mélange des radicaux

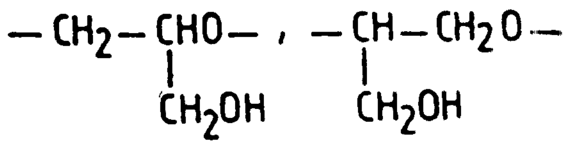

. $-OC_2H_3(R')-$ est constitué par un mélange des radicaux :

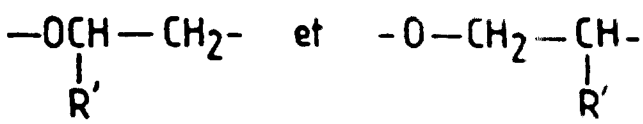

. $\bar{n}$ est une valeur statistique moyenne égale à 2,7 telle que déterminée par RMN $^1H$ à 500 MHz

.

| | |
|---|---|
| R' est un mélange des radicaux $C_{14}H_{23}$ et $C_{16}H_{33}$ | 1,61 g |
| - Cholestérol | 0,40 g |

On procède comme pour la première étape de l'exemple 1.

*Deuxième étape :*

On prélève le film lipidique formulé à l'issue de la première étape, et on le place dans un flacon de 60 ml. On ajoute aux lipides 2,03 g d'eau. On soumet le mélange obtenu au cycle suivant, répété deux fois : homogénéisation à l'aide d'une spatule - chauffage en étuve à 75°C pendant une heure - retour à la température ambiante par refroidissement spontané à l'air

*Troisième étape :*

On procède comme pour la troisième étape de l'exemple 1, excepté que l'on utilise 14,90 g d'une solution contenant 1,00 % de 5,6-dihydroxyindole et 0,42 % d'iodure de potassium dans l'eau.

*Quatrième étape :*

A la dispersion obtenue à l'issue de la troisième étape, on ajoute, sous agitation douce, 0,20 g d'une solution d'hydroxyde de sodium 0,1 M dans l'eau. Le pH de la dispersion est égal à 7,55.

*Cinquième étape :*

On procède comme pour la cinquième étape de l'exemple 1, excepté que l'on ajoute 0,90 g d'une solution de peroxyde d'hydrogène à 19,1 volumes dans l'eau.

La composition pondérale de la préparation ainsi réalisée est la suivante :

| | |
|---|---|
| - Lipide non-ionique de formule (IX) | 8,04 % |
| - Cholestérol | 2,01 % |
| - 5,6-dihydroxyindole polymérisé | 0,74 % |
| - Excipient aqueux qsp | 100 % |

La dimension moyenne des particules dispersées (vésicules et pigments), mesurée après 1 jour de conservation à température ambiante, est égale à 285 ± 4 nm ; et la dimension moyenne des pigments, mesurée après 8 jours de conservation à température ambiante, est égale à 193 ± 2 nm.

**EXEMPLE 6 :**

*Première étape :*

On utilise une phase lipidique formulée comme suit :

| | |
|---|---|
| - Lécithine hydrogénée, commercialisée par la | 2,15 g |

On introduit les constituants lipidiques précités dans un ballon de 100 ml, puis on les dissout à 50°C dans 20 ml de chloroforme et 5 ml de méthanol. Le solvant est ensuite évaporé à 40°C sous pression réduite, par paliers successifs, depuis la pression ambiante jusqu'à environ 500 Pa, à l'aide d'un évaporateur rotatif.

*Deuxième étape :*

On procède comme pour la deuxième étape de l'exemple 1, excepté que l'on utilise 2,89 g d'eau.

*Troisième étape :*

On procède comme pour la troisième étape de l'exemple 1, excepté que l'on utilise 20,85 g d'une solution contenant 1,00 % de 5,6-dihydroxyindole et 0,42 % d'iodure de potassium dans l'eau.

*Quatrième étape :*

A la dispersion obtenue à l'issue de la troisième étape, on ajoute, sous agitation douce, 1,50 g d'une solution d'hydroxyde de sodium 0,1 M dans l'eau Le pH de la dispersion est égal à 7,60.

*Cinquième étape :*

On procède comme pour la cinquième étape de l'exemple 1, excepté que l'on utilise 1,26 g d'une solution de peroxyde d'hydrogène à 19,1 volumes dans l'eau.

La composition pondérale de la préparation ainsi réalisée est la suivante :

| | |
|---|---|
| - Lécithine hydrogénée (Lecinol S10) | 7,32 % |
| - Cholestérol | 1,95 % |
| - Dihexadécylphosphate de sodium | 0,49 % |
| - 5,6-dihydroxyindole polymérisé | 0,71 % |
| - Excipient aqueux qsp | 100 % |

La fraction volumique des particules dispersées dans cette préparation (vésicules et pigments à base de 5,6-dihydroxyindole polymérisé) est égale à 59 % après 10 jours de conservation à température ambiante.

La dimension moyenne des particules dispersées (vésicules et pigments), mesurée après 10 jours de conservation à température ambiante est égale à 379 ± 11 nm ; et la dimension moyenne des pigments, mesurée après 21 jours de conservation à température ambiante, est égale à 291 ± 4 nm.

**EXEMPLE 7 :**

*Première étape :*

On utilise une phase lipidique formulée comme suit :

| | |
|---|---|
| - Lipide non-ionique de formule (VIII) | 0,58 g |
| - Alcool hexadécylique polyoxyéthyléné (20 motifs d'oxyde d'éthylène (OE) en moyenne), commercialisé par la Société "ICI ATLAS" sous la dénomination "BRIJ 58" | 0,55 g |
| - Cholestérol | 1,13 g |
| - Dihexadécylphosphate de sodium | 0,12 g |

On procède comme pour la première étape de l'exemple 1.

*Deuxième étape :*

On procède comme pour la deuxième étape de l'exemple 1, excepté que l'on utilise 2,37 g d'eau.

*Troisième étape :*

A la phase lamellaire issue de la deuxième étape, on ajoute 18,95 g d'une solution contenant 1,00 % de 5,6-dihydroxyindole, 0,2 % d'iodure de potassium dans l'eau. Le mélange ainsi obtenu est agité par secouage pendant 15 minutes à la température ambiante, chauffé à 30°C, puis traité pendant 8 minutes à l'aide d'un homogénéisateur à ultrasons.

*Quatrième étape :*

A la dispersion de vésicules obtenue à l'issue de la troisième étape, on ajoute 0,95 g d'une solution de peroxyde d'hydrogène à 20 volumes dans l'eau, et on agite l'ensemble par secouage pendant 15 minutes à la température ambiante.

La composition pondérale de la préparation ainsi réalisée est la suivante :

| | |
|---|---|
| - Lipide non-ionique de formule (VIII) | 2,43 % |
| - Alcool hexadécylique polyoxyéthyléné | 2,33 % |
| - Cholestérol | 4,75 % |
| - Dihexadécylphosphate de sodium | 0,50 % |
| - 5,6-dihydroxyindole polymérisé | 0,80 % |
| - Excipient aqueux qsp | 100 % |

La dimension moyenne des particules dispersées (vésicules et pigments), mesurée après 1 mois de conservation à température ambiante est égale à 307 ± 6 nm ; et la dimension moyenne des pigments, mesurée après 1 mois de conservation à température ambiante, est égale à 122 ± 1 nm

**<u>EXEMPLE 8</u> :**

*Première étape :*

On utilise une phase lipidique formulée comme suit :

| | |
|---|---|
| - Lipide non-ionique de formule (VIII) | 1,66 g |
| - Cholestérol | 1,66 g |
| - Dihexadécylphosphate de sodium | 0,17 g |

On introduit, dans un ballon de 100 ml, les constituants lipidiques précités, puis on les dissout dans 15 ml de dichlorométhane. Le solvant est ensuite évaporé à 40°C sous pression réduite, par paliers successifs, depuis la pression ambiante jusqu'à environ 400 Pa à l'aide d'un évaporateur rotatif.

*Deuxième étape :*

Le film lipidique formé à l'issue de la première étape est prélevé et placé dans un flacon de 60 ml. On ajoute aux lipides 3,53 g d'un mélange eau/éthanol à 95°, dans les proportions respectives 75/25. On soumet le mélange obtenu au cycle suivant, répété deux fois : homogénéisation à l'aide d'une spatule - chauffage en étuve à 75°C pendant 1 heure - retour à la température ambiante par refroidissement spontané à l'air.

*Troisième étape :*

On procède comme pour la troisième étape de l'exemple 1, excepté que l'on ajoute 22,20 g d'une solution contenant 1,21 % de 5,6-dihydroxyindole et 0,51 % d'iodure de potassium dans un mélange 75/25 eau/éthanol (à 95°)

*Quatrième étape :*

On procède comme pour la quatrième étape de l'exemple 1, excepté que l'on ajoute 0,50 g d'une solution d'hydroxyde de sodium à 0,1 M dans l'eau. Le pH de la dispersion est égal à 8,50.

*Cinquième étape :*

On procède comme pour la cinquième étape de l'exemple 1, excepté que lon ajoute 1,60 g d'une solution de peroxyde d'hydrogène à 19,9 volum es dans l'eau.

La composition pondérale de la préparation ainsi réalisée est la suivante :

| | |
|---|---|
| - Lipide non-ionique de formule (VIII) | 5,44 % |
| - Cholestérol | 5,44 % |
| - Dihexadécylphosphate de sodium | 0,56 % |
| - 5,6-dihydroxyindole polymérisé | 0,98 % |
| - Ethanol | 19,40 % |
| - Excipient aqueux qsp | 100 % |

La fraction volumique des particules dispersées dans cette préparation (vésicules et pigments à base de 5,6-dihydroxyindole polymérisé) est égale à 41 % après 1 jour de conservation à température ambiante.

La dimension moyenne des pigments, mesurée après 1 jour de conservation à température ambiante, est égale à 295 $\pm$ 6 nm.

**EXEMPLE 9 :**

*Première étape :*

On utilise une phase lipidique formulée comme suit :

| | |
|---|---|
| - Lécithine commercialisée par la Société "LUCAS MEYER" sous la dénomination "EPIKURON 200" | 3,04 g |
| - Cholestérol | 0,76 g |

On procède comme pour la première étape de l'exemple 1.

*Deuxième étape :*

Le film lipidique formé à l'issue de la première étape est prélevé et placé dans un flacon de 60 ml On ajoute aux lipides 3,80 g d'eau. Le mélange obtenu est soumis une fois au cycle suivant : homogénéisation à l'aide d'une spatule - chauffage en étuve à 75°C pendant 1 heure - retour à la température ambiante par refroidissement spontané à l'air.

*Troisième étape :*

A la phase lamellaire issue de la deuxième étape, on ajoute 23,58 g d'une solution de nitrate d'argent 0,05 M dans l'eau. Le mélange ainsi obtenu est agité par secouage pendant 30 minutes à la température ambiante, il est chauffé à 30°C, puis traité pendant 8 minutes à l'aide d'un homogénéisateur à ultrasons.

*Quatrième étape :*

A la dispersion de vésicules obtenue à l'issue de la troisième étape, on ajoute 1,30 g d'une solution d'hydroxyde de sodium 1 M dans l'eau à raison de 0,1 ml toutes les 30 secondes. A l'issue de l'addition de la solution d'hydroxyde de sodium précitée, le pH de la dispersion est voisin de 11. La dispersion est ensuite laissée au repos à température

ambiante.

La composition pondérale de la dispersion ainsi réalisée est la suivante :

| | |
|---|---|
| - Lécithine | 9,36 % |
| - Cholestérol | 2,33 % |
| - Oxyde d'argent ($Ag_2O$) | 0,42 % |
| - Excipient aqueux qsp | 100 % |

La dimension moyenne des particules dispersées (vésicules et pigments d'oxyde d'argent), mesurée après un jour de conservation à température ambiante, est égale à 190 ± 3 nm ; et la dimension moyenne des pigments d'oxyde d'argent, mesurée après un jour de conservation à température ambiante, est égale à 133 ± 1 nm.

**<u>EXEMPLE 10</u> :**

*Première étape :*

On utilise une phase lipidique formulée comme suit

| | |
|---|---|
| - Lipide non-ionique de formule (VIII) | 1,43 g |
| - Cholestérol | 1,43 g |
| - Dihexadécylphosphate de sodium | 0,15 g |

On introduit les constituants précités dans un ballon de 100 ml, puis on les dissout dans 5 ml de dichlorométhane. Ensuite, le solvant est évaporé à 40°C sous pression réduite, par paliers successifs, depuis la pression ambiante jusqu'à environ 400 Pa, à l'aide d'un évaporateur rotatif.

*Deuxième étape :*

On prélève le film lipidique formé à l'issue de la première étape, et on le place dans un flacon de 15 ml. On ajoute aux lipides 3,02 g d'eau. On soumet le mélange obtenu au cycle suivant, lequel est répété 6 fois : homogénéisation à l'aide d une spatule - chauffage en étuve à 75°C pendant 30 mn - retour à la température ambiante par refroidissement spontané à l'air.

*Troisième étape :*

A la phase lamellaire issue de ta deuxième étape, on ajoute 17,14 g d'une solution contenant 1,73 % de bromhydrate de 5,6-dihydroxyindoline dans l'eau. Le mélange ainsi obtenu est agité par secouage, pendant 15 mn, à la température ambiante.

*Quatrième étape :*

A la dispersion obtenue à l'issue de la troisième étape, on ajoute, sous agitation douce, 1,3 ml d'une solution d'hydroxyde de sodium 1 M dans l'eau. Le pH de la dispersion est égal à 7,50.

*Cinquième étape :*

A la dispersion obtenue à l'issue de la quatrième étape, on ajoute 1,45 g d'une solution de peroxyde d'hydrogène à 20 volumes dans l'eau puis 5,21 g d'eau. Le mélange est traité pendant 9 minutes à l'aide d'un homogénéisateur à ultrasons.

*Sixième étape :*

La dispersion est ensuite chauffée pendant 4 heures à 80°C sous agitation douce, puis laissée au repos à température ambiante.

La dimension moyenne des particules dispersées (vésicules et pigments), mesurée après 1 jour de conservation à températrue ambiante, est égale à 1140 ± 160 nm ; et la dimension moyenne des pigments, mesurée après 1 jour de conservation à température ambiante, est égale à 250 ± 6 nm.

**EXEMPLE 11 :**

*Première étape :*

On utilise une phase lipidique formulée comme suit :

| | |
|---|---|
| - Lipide non-ionique de formule (VIII) | 0,72 g |
| - Alcool hexadécylique polyoxyéthyléné (20 motifs de OE en moyenne), commercialisé sous la dénomination "BRIJ 58" par la Société "ICI ATLAS" | 0,72 g |
| - Cholestérol | 1,45 g |
| - Dihexadécylphosphate de sodium | 0,15 g |

On introduit les constituants précités dans un ballon de 100 ml, puis on les dissout dans 6 ml de dichlorométhane. Ensuite, le solvant est évaporé à 40°C sous pression réduite, par paliers successifs, depuis la pression ambiante jusqu'à environ 400 Pa, à l'aide d'un évaporateur rotatif.

*Deuxième étape :*

On prélève le film lipidique formé à l'issue de la première étape, et on le place dans un flacon de 30 ml. On ajoute aux lipides 3,05 g d'eau. On soumet le mélange obtenu au cycle suivant, lequel est répété 4 fois : homogénéisation à l'aide d'une spatule - chauffage en étuve à 75°C pendant 30 mn - retour à la température ambiante par refroidissement spontané à l'air.

*Troisième étape :*

A la phase lamellaire issue de la deuxième étape, on ajoute 17,3 g d'une solution contenant 0,30 g de bromhydrate d'indoline et 17 g d'eau.

Le mélange ainsi obtenu est agité pendant 15 minutes, à la température ambiante, à l'aide d'un agitateur à hélice.

*Quatrième étape :*

La dispersion obtenue à l'issue de la troisième étape est traitée pendant 6 mn à l'aide d'un homogénéisateur à ultrasons.

*Cinquième étape :*

Le pH de la dispersion est ajusté à 11,8 par addition de 1,3 ml d'une solution d'hydroxyde de sodium 1 M dans l'eau.

On ajoute ensuite 1,45 g de solution de peroxyde d'hydrogène à 20 volumes dans l'eau puis 3,85 g d'eau, sous agitation douce.

*Sixième étape :*

La dispersion obtenue est traitée pendant 2 mn à l'aide d'un homogénéisateur à ultrasons

*Septième étape :*

La dispersion est agitée à l aide d'une secoueuse à bras oscillants pendant 2 heures à la température de 80°C puis laissée au repos à température ambiante.

La dimension moyenne des particules dispersées (vésicules et pigments), mesurée après 1 jour de conservation à température ambiante est égale à 294 ± 2 nm ; et la dimension moyenne des pigments, mesurée après 1 jour de conservation à température ambiante, est égale à 235 ± 40 nm.

**Revendications**

1. Procédé de préparation de particules submicroniques en présence de vésicules lipidiques, à partir d'au moins un précurseur desdites particules, à partir d'au moins un lipide et d'une première phase aqueuse dont une partie est destinée à être encapsulée dans les vésicules et, éventuellement une deuxième phase aqueuse destinée à faire partie du milieu de dispersion desdites vésicules, caractérisé par le fait qu'on ajoute au moins un précurseur desdites particules dans la première phase aqueuse et/ou la deuxième phase aqueuse, avant et/ou pendant et/ou après la préparation de la phase vésiculaire, au moins un agent capable de transformer ledit (ou lesdits) précurseur(s) en les particules recherchées étant amené à agir sur celui-ci (ou ceux-ci) après son (ou leur) introduction dans le milieu de préparation, le précurseur contenu dans la phase aqueuse de dispersion n'étant pas éliminé avant l'introduction dudit agent de façon que la phase aqueuse de dispersion contienne des particules précipitées submicroniques.

2. Procédé selon la revendication 1, caractérisé par le fait que l'on définit l'homogénéisation et l'agitation pour obtenir des vésicules ayant des dimensions comprises entre 20 et 3000 nm.

3. Procédé selon l'une des revendications 1 ou 2, caractérisé par le fait qu'on prépare une phase lamellaire dans laquelle la concentration en lipide(s) est comprise entre 10 et 90% en poids.

4. Procédé selon l une des revendications 1 à 3, caractérisée par le fait qu'on prépare une phase vesiculaire dans laquelle la concentration en lipides est comprise entre 1 et 90 % en poids.

5. Procédé selon l une des revendications 1 à 4. caractérisé par le fait qu'on prépare la phase vésiculaire a une température comprise entre 10 et 150°C.

6. Procédé selon l'une des revendications 1 à 5, caractérisé par le fait qu'on utilise au moins un précurseur susceptible de précipiter en particules submicroniques sous l'action d'au moins un agent de précipitation.

7. Procédé selon l'une des revendications 1 à 5. caractérisé par le fait qu'on utilise, comme precurseur, au moins un monomère polymérisable, sur lequel ou fait agir au moins un agent de polymérisation.

8. Procédé selon l'une des revendications 1 à 7, caractérisé par le fait qu'on conduit la transformation du (ou des) précurseur(s) après la formation des vésicules.

9. Procédé selon l'une des revendications 1 à 8, caractérisé par le fait qu'on choisit le (ou les) lipide(s) parmi les lipides amphiphiles non-ioniques, les lipides amphiphiles ioniques et les mélanges de lipides non-ionique(s) et de lipides ioniques.

10. Procédé selon la revendication 9, caractérise par le fait qu'on choisit les lipides amphiphiles non-ioniques dans le groupe formé par :

    (1) les dérivés de polyglycérol linéaires ou ramifiés de formule :

$$R^0O-\left[C_3H_5(OH)O\right]_{\bar{n}}-H \qquad (I)$$

    formule dans laquelle :

. $-C_3H_5(OH)O$ est représente par les structures suivantes prises en mélange ou séparément :

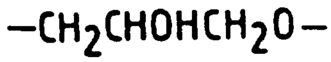 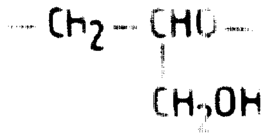 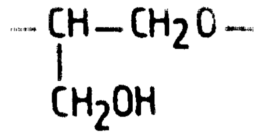

. $\overline{n}$ est une valeur statistique moyenne comprise entre 1 et 6 ;

. $R^o$ représente :

(a) une chaîne aliphatique, linéaire ou ramifiée , saturée ou insaturée, contenant de 12 à 30 atomes de carbone ; ou des radicaux hydrocarbonés des alcools de lanoline ; ou les restes d'alpha-diols à longue chaîne ;

(b) un reste $R^1CO$ ou $R^1$ est un radical aliphatique, linéaire ou ramifié, en $C_{11}$-$C_{29}$ ;

(c) un reste $R^2$-[-$OC_2H_3(R^3)$-]- , où :

. $R^2$ peut prendre la signification (a) ou (b) donnée pour $R^o$ ;

. $OC_2H_3(R^3)$- est représente par les structures suivantes, prises en mélange ou séparément :

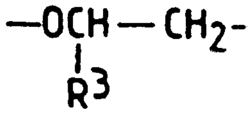 et 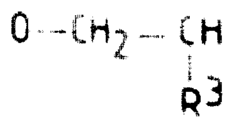

où $R^3$ prend la signification (a) donnée pour $R^o$ ;

(2) les éthers de polyglycérol , linéaires ou ramifiés, comportant deux chaînes grasses ;
(3) les alcools gras polyoxyéthylénés et les stérols et phytostérols polyoxyéthylénés ;
(4) les éthers de polyols ;
(5) les esters de polyols, oxyéthylénés ou non ;
(6) les glycolipides d'origine naturelle ou synthétique et notamment les éthers et les esters de glucose ;
(7) les hydroxyamides représentés par la formule (II) :

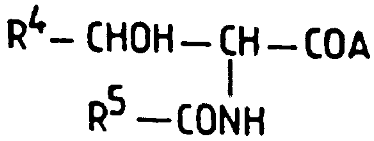 (II)

formule dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :

. un reste

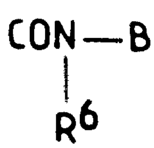

où :

- B est un radical alcoyle dérivé d'amines primaires ou secondaires, mono- ou polyhydroxylées ; et
- $R^6$ désigne un atome d'hydrogène ou un radical méthyle, éthyle ou hydroxyéthyle ; et

- un reste -COOZ, où Z représente le reste d'un polyol en $C_3$-$C_7$.

(8) les dérivés du glycérol répondant à la formule :

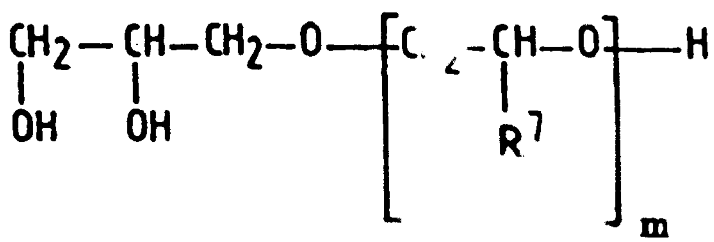

(III)

formule dans laquelle $R^7$ représente un radical alkyle linéaire en $C_{14}$ à $C_{18}$ ou un groupement $-CH_2A$ dans lequel A est $-OR_{14}$, $R_{14}$ représentant un radical alkyle linéaire en $C_{10}$-$C_{18}$ et, de préférence, en $C_{16}$, et m représente une valeur statistique moyenne supérieure à 1 et au plus égale à 3 et, en outre, lorsque $R_7$ = $-CH_2A$, m peut également représenter une valeur réelle (non statistique) égale à 2.

(9) les esters de glucose de formule :

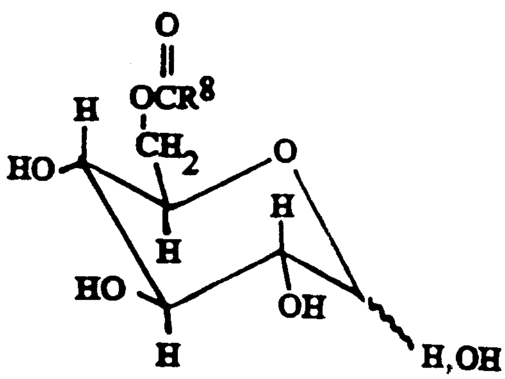

(IV)

formule dans laquelle $R^8$ représente une chaîne hydrocarbonée, linéaire, saturée ou insaturée, contenant de 9 à 17 atomes de carbone.

**11.** Procédé selon la revendication 9, caractérisé par le fait qu'on choisit les lipides amphiphiles ioniques dans la groupe formé : par les phospholipides naturels , les phospholipides de synthèse; les composés anioniques ; les composés cationiques dérivés ammonium quaternaire répondant à la formule :

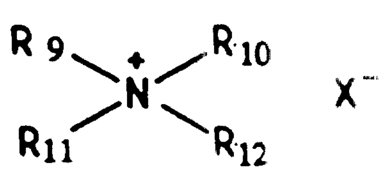

(V)

formule dans laquelle $R_9$ et $R_{10}$, identiques ou différents, représentent des radicaux alkyle en $C_{12}$-$C_{20}$ et $R_{11}$ et $R_{12}$ , identiques ou différents, représentent des radicaux alkyle en $C_1$-$C_4$ ; les lipides polymérisables.

**12.** Procédé selon l'une des revendications 9 à 11, caractérisé par le fait qu'on associe aux lipides des additifs choisis dans le groupe formé par:

- les alcools et diols à longue chaîne ;
- les stérols ;
- les amines à longue chaîne et leurs dérivés ammonium quaternaire ;
- les dihydroxyalkylamines ; les amines grasses polyoxyéthylénées ; les esters d'amino-alcools à longue chaîne ; et leurs sels et dérivés ammonium quaternaire ;
- les esters phosphoriques d'alcools gras les alkylsulfates ; et
- les polypeptides et les protéines.

**13.** Procédé selon lune des revendications 9 à 12, caractérisé par le fait qu'on associe à la phase lipidique propre des vésicules au moins un lipoprotide exempt de fonction sulfhydryle choisi parmi les dérivés mono- ou polyacylés d'amino-acides ou de polypeptides, dans le reste acyle $R_{13}$-CO comporte une chaîne hydrocarbonée $R_{13}$ en $C_{13}$-$C_{19}$, au moins une des fonctions qui relie la chaîne polypeptidique ou le reste d'amino-acide à la chaîne lipophile étant une fonction amide, les fonctions carboxyliques de la chaîne polypeptidique ou du reste d'amino-acide pouvant être partiellement ou totalement neutralisées par un ou plusieurs cations alcalins, un ion ammonium ou un ammonium substitué dérivé d'une amine, ledit (ou lesdits lipoprotides) étant présent(s) à raison de 1 à 15 % en poids par rapport au poids total de la phase lipidique propre, et/ou au moins un cholestérol-sulfate de cation ammonium, alcalin ou alcalino-terreux, à raison de 1 à 50 % en poids par rapport au poids de ladite phase lipidique propre et/ou au moins un cholestérol-phosphate, sous forme acide libre ou neutralisée par un cation ammonium, alcalin ou alcalino-terreux, à raison de 1 à 40 % en poids par rapport au poids total de la phase lipidique propre.

**14.** Procédé selon lune des revendications 1 à 13, caractérisé par le fait qu'on utilise, comme phase aqueuse E ou D, de l'eau ou un mélange d'eau et d'au moins un solvant miscible à l'eau.

**15.** Procédé selon la revendication 14, caractérisé par le fait qu'on choisit le (ou les) solvant(s) miscible(s) à l'eau parmi les alcohols en $C_1$-$C_7$ et les polyols d'alkyles en $C_1$-$C_5$.

**16.** Procédé selon l'une des revendications 1 à 15, dans lequel on prépare des particules submicroniques constituées par des pigments ou des polymères, caractérisé par le fait que l'on utilise des agents de précipitation ou de polymérisation compatibles avec les vésicules sur les plans chimique et physico-chimique et choisis dans le groupe formé par les oxydants, les initiateurs de radicaux libres, les enzymes, les radiations, les acides et les bases.

**17.** Procédé selon la revendication 16 dans lequel on prépare des particules submicroniques de pigments, caractérisé par le fait qu'on choisit, comme précurseur, au moins un composé indolique et/ou au moins un composé indolinique que l'on soumet à une oxydation ou à une polymérisation oxydante ou enzymatique.

**18.** Procédé selon la revendication 17, caractérisé par le fait qu'on choisit, comme précurseur, au moins un composé indolique répondant à la formule :

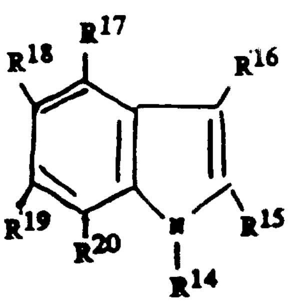

(VI)

formule dans laquelle :

- $R^{14}$ et $R^{16}$ représentent, indépendamment l'un de l'autre, l'hydrogène ou un radical alkyle en $C_1$-$C_4$ ;
- $R^{15}$ représente l'hydrogène, ou un radical alkyle en $C_1$-$C_4$, un groupe carboxyle ou (alcoxy en $C_1$-$C_4$)-carbonyle ;
- $R^{17}$ et $R^{20}$ représentent, indépendamment l'un de l'autre, l'hydrogène, un groupe hydroxy, un groupe alkyle en $C_1$-$C_4$, un groupe amino, alcoxy en $C_1$-$C_4$, (alcoxy en $C_1$-$C_4$)-oxy, (acyl en $C_2$-$C_4$)-amino ;
- $R^{18}$ représente l'hydrogène, un groupe hydroxy, alcoxy en $C_1$-$C_4$, un radical alkyle en $C_1$-$C_4$, un groupe halogène, amino, (acyl en $C_2$-$C_4$)-oxy, (acyl en $C_2$-$C_4$)-amino, triméthylsilyloxy ;

- $R^{19}$ représente l'hydrogène, un groupe hydroxy, alcoxy en $C_1$-$C_4$, amino, (acyl en $C_2$-$C_4$)-oxy, (acyl en $C_2$-$C_4$)-amino, triméthylsilyloxy, (hydroxyalkyl en $C_2$-$C_4$)-amino ;
- $R^{18}$ et $R^{19}$ pouvant également former, conjointement avec les atomes de carbone auxquels ils sont rattachés, un cycle méthylènedioxy éventuellement substitué par un groupement alkyle en $C_1$-$C_4$ ou alcoxy en $C_1$-$C_4$, ou bien un cycle carbonyldioxy ;

  avec la condition qu'au moins l'un des radicaux $R^{17}$ à $R^{20}$ représente un groupement OZ ou $NHR^{21}$ avec au plus un des radicaux $R^{17}$ à $R^{20}$ représentant $NHR^{21}$ ; et au plus deux des radicaux $R^{17}$ à $R^{20}$ représentent OZ et, dans le cas où Z représente l'hydrogène, les deux OH sont dans les positions 5 et 6 ; et au moins l'un des radicaux $R^{17}$ à $R^{20}$ représente l'hydrogène, et dans le cas où un seul de ces radicaux représente l'hydrogène, un seul radical parmi $R^{17}$ à $R^{20}$ représente alors $NHR^{21}$ ou OZ, les autres radicaux représentant un radical alkyle en $C_1$-$C_4$ ; $R^{21}$ désignant l'hydrogène, un groupe acyle en $C_2$-$C_4$, hydroxyalkyle en $C_2$-$C_4$, et Z désignant l'hydrogène, un groupe acyle en $C_2$-$C_{14}$, alkyle en $C_1$-$C_4$, ou triméthylsilyle ;

et/ou au moins un de leurs sels de métaux alcalins, alcalino-terreux, d'ammonium ou d'amines, et de leurs chlorhydrates, bromhydrates, sulfates et méthane sulfonates.

**19.** Procédé selon la revendication 18, caractérisé par le fait qu'on choisit le composé indolique dans le groupe formé par le 4-hydroxyindole, le 5-hydroxyindole, le 6-hydroxyindole, le 7-hydroxyindole, le 4-hydroxy 5-méthoxyindole, le 4-hydroxy 5-éthoxyindole, le 2-carboxy 5-hydroxyindole, le 5-hydroxy 6-méthoxyindole , le 6-hydroxy 7-méthoxyindole, le 5-méthoxy 6-hydroxyindole, le 5,6-dihydroxyindole, le N-méthyl 5,6-dthydroxyindole, le 2-méthyl 5,6-hydroxyindole, le 3-méthyl 5,6-bydroxyindole, le 2,3-diméthyl 5,6-dihydroxyindole, le 2-carboxyl 5,6-dihydroxyindole, le 4-hydroxy 5-méthyl indole, le 2-carboxyl 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7 hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxyl 5-méthyl-indole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6 - hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole et le 5,6-diméthoxyindole, et les sels d'addition de ces composés.

**20.** Procédé selon la revendication 17, caractérisée par le fait qu'on choisit, comme précurseur, au moins un composé indolinique de formule :

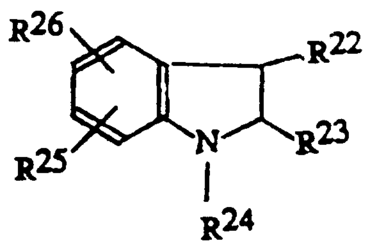

(VII)

formule dans laquelle :

- $R^{22}$ et $R^{24}$ représentent, indépendamment l'un de l'autre, un atome d'hydrogène ou un radical alkyle en $C_1$-$C_4$ ;
- $R^{23}$ représente un atome d'hydrogène, un radical alkyle en $C_1$-$C_4$, ---------- un groupe carboxyle ou alcoxy ($C_1$-$C_4$) carbonyl ;
- $R^{25}$ désigne un atome d'hydrogène, un radical alkyle en $C_1$-$C_4$, hydroxyle, alcoxy ($C_1$-$C_4$), amino ou alkylamino en $C_1$-$C_{10}$ ou halogène ;
- $R^{26}$ désigne un atome d'hydrogène, un groupe hydroxyle, alcoxy en $C_1$-$C_4$ ou amino ; avec la condition qu'au moins un des radicaux $R^{25}$ ou $R^{26}$ désigne un groupe hydroxyle, alcoxy ou amino ; et sous réserve que lorsque $R^{26}$ désigne un groupe amino, $R^{25}$ ne peut désigner un radical alkylamino ;
- $R^{25}$ et $R^{26}$ pouvant également former un cycle alkylènedioxy en $C_1$-$C_2$, lorsqu'ils sont en position 5 et 6 ; et/ou au moins un de leurs sels de métaux alcalins, alcalino-terreux d'ammonium ou d'amines, et de leurs chlorydrates, bromhydrates, sulfates et méthanesulfonates.

21. Procédé selon la revendication 20, caractérisé par le fait que l'on choisit le composé indolinique dans le groupe formé par la 5,6-dihydroxyoindoline, la 6-hydroxyindoline, la 5,6-méthylènedioxyindoline, la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6-aminoindoline, la 5,6-diaminoindoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amino 6-chloroindoline, la 4-amino 6-iodoindoline, la 4-amino 5-bromoindoline, la 4-amino 5-dydroxyindoline, la 4-amino 7-hydroxyindoline, la 4-amino 5-méthoxyindoline, la 4-amino 7-méthoxyindoline, la 5-aminoindoline, la 2,3-diméthyl 5-aminoindoline, la 1-méthyl 5-aminoindoline, la 2-méthyl 5-aminoindoline, la 5-N-(1-méthythexyl)aminoindoline, la 5,6-diméthoxyindoline et la 5,6-dihydroxy 2-carboxyindoline.

22. Procédé selon la revendication 16, caractérisé par le fait qu'on prépare des particules de polymères d'acrylamide et de bisacrylamides.

23. Procédé selon l'une des revendications 1 à 16, caractérisé par le fait que les particules submicroniques sont des oxydes métalliques.

24. Procédé selon la revendication 23, caractérisé par le fait que les particules submicroniques sont des oxydes d'argent, de fer ou de zinc.

25. Procédé selon lune des revendications 1 à 16, caractérisé par le fait que les particules submicroniques sont des sulfures métalliques.

26. Procédé selon la revendication 25, caractérisé par le fait que les particules submicroniques sont des sulfures de cadmium, zinc, plomb ou cuivre.

27. Procédé selon l'une des revendications 1 à 22, caractérisé par le fait que, pour préparer la phase lamellaire, on solubilise le(s) lipide(s) devant former la paroi des vésicules dans un solvant, on fait évaporer le solvant sous une pression réduite, puis on mélange l'association lipidique ainsi formée avec la phase aqueuse E, on homogénéise, on chauffe à une température comprise entre 10 et 150°C pendant un temps compris entre 0,25 et 2 h et on fait revenir à la température ambiante, en réitérant au moins une fois le cycle homogénéisation - chauffage - retour à la température ambiante.

28. Procédé selon la revendication 27, caractérisé par le fait qu'on utilise, comme solvant, le dichlorméthane, le chloroforme, l'acétate d'éthyle, l'acétate de butyle, le formiate d'éthyle, l'hexane, le cyclohexane, le toluène, l'éther de pétrole, le méthanol, l'éthanol, le propanol, l'éther méthylique, l'éther éthylique, et les mélanges d'au moins deux d'entre eux.

29. Composition obtenue par le procédé selon l'une des revendications 1 à 28.

30. Composition selon la revendication 29 comportant un milieu aqueux où sont dispersées des vésicules lipidiques et des particules submicroniques, lesdites particules étant contenues dans lesdites vésicules et/ou indépendantes de celles-ci, caractérisée par le fait que le volume desdites vésicules et, le cas échéant, des particules indépendantes de celles-ci, représente 5 à 90 % du milieu, les lipides vésiculaires représentant de 1 à 90 % en volume par rapport au volume total de la composition, les vésicules ayant des dimensions comprises entre 20 et 3000 nm.

31. Composition selon l'une des revendications 29 ou 30, caractérisée par le fait qu'elle contient, en milieu aqueux de 0,1 à 20 % en poids de particules de pigments ayant des dimensions comprises entre 20 et 500 nm.

## Claims

1. Method for preparing submicron particles in the presence of lipid vesicles, from at least one precursor of the said particles, from at least one lipid and from a first aqueous phase, part of which is intended to be encapsulated into the vesicles and, optionally, a second agueous phase intended to form part of the medium for dispersing the said vesicles, characterized in that at least one precursor of the said particles is added to the first aqueous phase and/or the second aqueous phase, before and/or during and/or after the preparation of the vesicular phase, at least one

agent capable of converting the said precursor(s) to the desired particles being made to act on it (or on them) after its (or their) introduction into the preparation medium, the precursor contained in the aqueous dispersion phase not being eliminated before the introduction of the said agent so that the aqueous dispersion phase contains submicron precipitated particles.

**2.** Method according to Claim 1, characterized in that the homogenization and the stirring are defined so as to obtain vesicles having sizes of between 20 and 3000 nm.

**3.** Method according to either of Claims 1 and 2, characterized in that a lamellar phase is prepared in which the concentration of lipid(s) is between 10 and 90% by weight.

**4.** Method according to one of Claims 1 to 3, characterized in that a vesicular phase is prepared in which the concentration of lipids is between 1 and 90% by weight.

**5.** Method according to one of Claims 1 to 4, characterized in that the vesicular phase is prepared at a temperature of between 10 and 150°C

**6.** Method according to one of Claims 1 to 5, characterized in that at least one precursor capable of precipitating as submicron particles under the action of at least one precipitating agent is used.

**7.** Method according to one of Claims 1 to 5, characterized in that at least one polymerizable monomer, on which at least one polymerizing agent is caused to react, is used as precursor.

**8.** Method according to one of Claims 1 to 7, characterized in that the conversion of the precursor(s) is performed after the formation of the vesicles.

**9.** Method according to one of Claims 1 to 8, characterized in that the lipid(s) is (are) chosen from non-ionic amphiphilic lipids, ionic amphiphilic lipids, and mixtures of nonionic lipids and ionic lipids.

**10.** Method according to Claim 9, characterized in that the nonionic amphiphilic lipids are chosen from the group consisting of:

(1) the linear or branched polyglycerol derivatives of formula:

$$R^0O-\left[C_3H_5(OH)O\right]_{\overline{n}}-H \qquad (I)$$

in which formula:

. $-C_3H_5(OH)O$ is represented by the following structures taken as a mixture or separately:

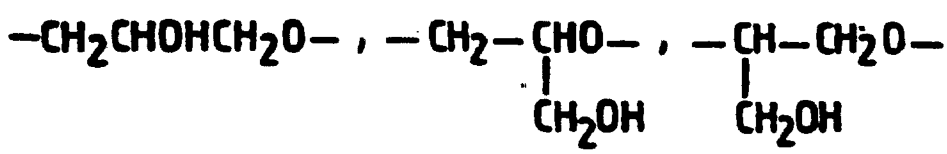

. $\overline{n}$ is a mean statistical value between 1 and 6;
. $R^0$ represents:

(a) a saturated or unsaturated, linear or branched aliphatic chain containing from 12 to 30 carbon atoms; or hydrocarbon-containing radicals of lanolin alcohols; or long-chain alpha-diol residues;
(b) a residue $R^1CO$, where $R^1$ is a linear or branched $C_{11}$-$C_{29}$ aliphatic radical,
(c) a residue $R^2$-[-$OC_2H_3(R^3)$-]-, where:

. $R^2$ may have the meaning (a) or (b) given for $R^0$;

. $OC_2H_3(R^3)$- is represented by the following structures, taken as a mixture or separately:

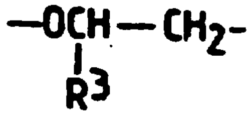

and

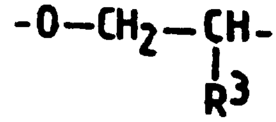

where $R^3$ has the meaning (a) given for $R^0$;

(2) the linear or branched polyglycerol ethers containing two fatty chains;
(3) the polyoxyethylenated fatty alcohols and the polyoxyethylenated sterols and phytosterols;
(4) the polyol ethers;
(5) the polyol esters, oxyethylenated or otherwise;
(6) the glycolipids of natural or synthetic origin, and in particular the glucose ethers and esters;
(7) the hydroxyamides represented by the formula (II):

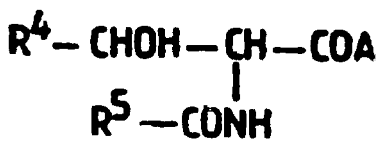

(II)

in which formula:

- $R^4$ denotes a $C_7$-$C_{21}$ alkyl or alkenyl radical;
- $R^5$ denotes a saturated or unsaturated $C_7$-$C_{31}$ hydrocarbon radical;
- COA denotes a group chosen from the following two groups:

  . a residue

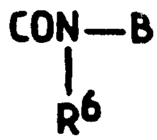

  where:

  . B is an alkyl radical derived from mono- or polyhydroxylated primary or secondary amines; and
  . $R^6$ denotes a hydrogen atom or a methyl, ethyl or hydroxyethyl radical; and
  . a residue -COOZ, where Z represents the residue of a $C_3$-$C_7$ polyol.

(8) the glycerol derivatives corresponding to the formula:

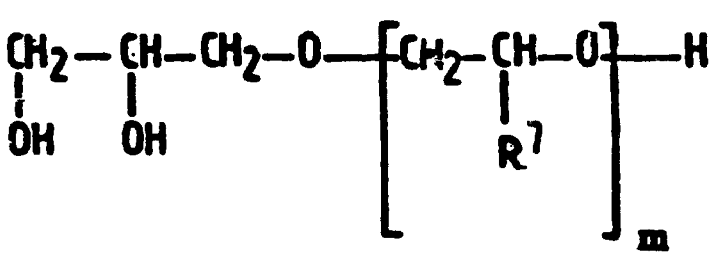

(III)

in which formula $R^7$ represents a linear $C_{14}$ to $C_{18}$ alkyl radical or a group $-CH_2A$ in which A is $-OR_{14}$, $R_{14}$ representing a linear $C_{10}$-$C_{18}$ and, preferably, $C_{16}$ alkyl radical, and m represents a mean statistical value greater

than 1 and at most equal to 3 and, in addition, when $R_7$ [sic] = $-CH_2A$, m may also represent a real (non statistical) value equal to 2.

(9) the glucose esters of formula:

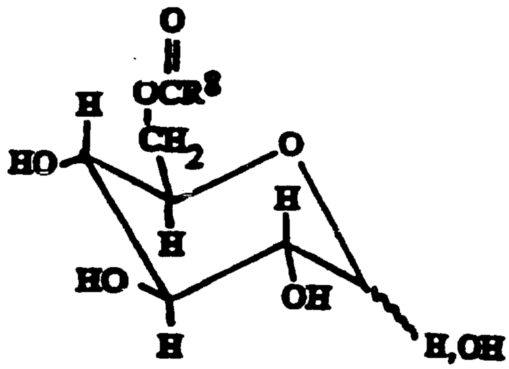

(IV)

in which formula $R^8$ represents a saturated or unsaturated linear hydrocarbon chain containing from 9 to 17 carbon atoms.

**11.** Method according to Claim 9, characterized in that the ionic amphiphilic lipids are chosen from the group consisting of: natural phospholipids; synthetic phospholipids; anionic compounds; quaternary ammonium-derived cationic compounds corresponding to the formula:

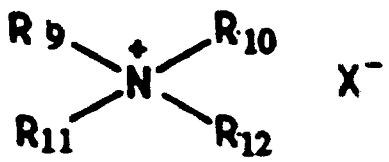

(V)

in which formula $R_9$ and $R_{10}$, which are identical or different, represent $C_{12}$-$C_{20}$ alkyl radicals and $R_{11}$ and $R_{12}$, which are identical or different, represent $C_1$-$C_4$ alkyl radicals; polymerizable lipids

**12.** Method according to one of Claims 9 to 11, characterized in that there are associated with the lipids additives chosen from the group consisting of:

- long-chain alcohols and diols;
- sterols;
- long-chain amines and their quaternary ammonium derivatives;
- dihydroxyalkylamines; polyoxyethylenated fatty amines; esters of long-chain amino alcohols; and their quaternary ammonium derivatives and salts;
- phosphoric esters of fatty alcohols; alkyl sulphates; and
- polypeptides and proteins

**13.** Method according to one of Claims 9 to 12, characterized in that there are associated with the actual lipid phase of the vesicles at least one lipoprotein free of sulphydryl functional group, chosen from the mono- or polyacylated derivatives of amino acids or polypeptides, in which the acyl residue $R_{13}$-CO contains a $C_{13}$-$C_{19}$ hydrocarbon chain $R_{13}$, at least one of the functional groups which links the polypeptide chain or the amino acid residue to the lipophilic chain being an amide functional groups, it being possible for the carboxyl functional groups of the polypeptide chain or of the amino acid residue to be partially or completely neutralized by one or more alkali metal cations, an ammonium ion or a substituted ammonium derived from an amine, the said lipoprotein(s) being present in an amount of 1 to 15% by weight relative to the total weight of the actual lipid phase; and/or at least one ammonium, alkali metal or alkaline-earth metal cation chlolesterol sulphate in an amount of 1 to 50% by weight relative to the weight of the said actual lipid phase and/or at least one cholesterol phosphate, in free acid form or in a form neutralized with an ammonium alkali metal or alkaline-earth metal cation, in an amount of 1 to 40% by weight relative to the total weight of the actual lipid phase.

**14.** Method according to one of Claims 1 to 13, characterized in that water or a mixture of water and of at least one water-miscible solvent is used as aqueous phase E or D.

**15.** Method according to Claim 14, characterized in that the water-miscible solvent(s) is (are) chosen from $C_1$-$C_7$ alcohols and $C_1$-$C_5$ alkyl polyols.

**16.** Method according to one of Claims 1 to 15, in which submicron particles consisting of pigments or polymers are prepared, characterized in that precipitating or polymerizing agents compatible with the vesicles from the chemical and physicochemical point of view and chosen from the group consisting of oxidants, initiators of free radicals, enzymes, radiation, acids and bases, are used.

**17.** Method according to Claim 16, in which submicron particles of pigments are prepared, characterized in that at least one indole compound and/or at least one indoline compound which is subjected to oxidation or to oxidative or enzymatic polymerization is chosen as precursor.

**18.** Method according to Claim 17, characterized in that there is chosen, as precursor, at least one indole compound corresponding to the formula:

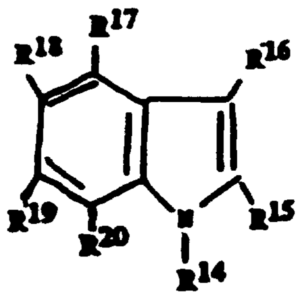

(VI)

in which formula:

- $R^{14}$ and $R^{16}$ represent, independently of each other, hydrogen or a $C_1$-$C_4$ alkyl radical;
- $R^{15}$ represents hydrogen, or a $C_1$-$C_4$ alkyl radical, a carboxyl or ($C_1$-$C_4$ alkcoxy)carbonyl group;
- $R^{17}$ and $R^{20}$ represent, independently of each other, hydrogen, a hydroxyl group, a $C_1$-$C_4$ alkyl group, an amino group, a $C_1$-$C_4$ alkoxy group, a ($C_1$-$C_4$ alkoxy)oxy group, or a ($C_2$-$C_4$ acyl)amino group;
- $R^{18}$ represents hydrogen, a hydroxyl group, a $C_1$-$C_4$ alkoxy group, a $C_1$-$C_4$ alkyl radical, a halogen group, an amino group, a ($C_2$-$C_4$ acyl)oxy group, a ($C_2$-$C_4$ acyl)amino group, or a trimethylsilyloxy group;
- $R^{19}$ represents hydrogen, a hydroxyl group, a $C_1$-$C_4$ alkoxy group, an amino group, a ($C_2$-$C_4$ acyl)oxy group, a ($C_2$-$C_4$ acyl)amino group, a trimethylsilyloxy group, a ($C_2$-$C_4$ hydroxyalkyl)amino group;
- it being possible for $R^{18}$ and $R^{19}$ to form, together with the carbon atoms to which they are attached, a methylenedioxy ring optionally substituted with a $C_1$-$C_4$ alkyl or $C_1$-$C_4$ alkoxy group or a carbonyldioxy ring;
  on the condition that at least one of the radicals $R^{17}$ to $R^{20}$ represents a group OZ or $NHR^{21}$ with at most one of the radicals $R^{17}$ to $R^{20}$ representing $NHR^{21}$; and at most two of the radicals $R^{17}$ to $R^{20}$ represent OZ and, in the case where Z represents hydrogen, the two OH groups are in the 5 and 6 positions; and at least one of the radicals $R^{17}$ to $R^{20}$ represents hydrogen, and in the case where only one of these radicals represents hydrogen, only one radical among $R^{17}$ to $R^{20}$ then represents $NHR^{21}$ or OZ, the other radicals representing a $C_1$-$C_4$ alkyl radical; $R^{21}$ denoting hydrogen, a $C_2$-$C_4$ acyl group, a $C_2$-$C_4$ hydroxyl alkyl, and Z denoting hydrogen, a $C_2$-$C_{14}$ acyl group, a $C_1$-$C_4$ alkyl group or a trimethylsilyl group;

and/or at least one of their alkali metal, alkaline-earth metal, ammonium or amine salts, and their hydrochlorides, hydrobromides, sulphates and methane sulphonates.

**19.** Method according to Claim 18, characterized in that the indole compound is chosen from the group consisting of 4-hydroxyindole, 5-hydroxyindole, 6-hydroxyindole, 7-hydroxyindole, 4-hydroxy-5-methoxyindole, 4-hydroxy-5-ethoxyindole, 2-carboxy-5-hydroxyindole, 5-hydroxy-6-methoxyindole, 6-hydroxy-7-methoxyindole, 5-methoxy-6-hydroxyindole, 5,6-dihydroxyindole, N-methyl-5,6-dihydroxyindole, 2-methyl-5,6-hydroxyindole, 3-methyl-5,6-hydroxyindole, 2,3-dimethyl-5,6-dihydroxyindole, 2-carboxyl-5,6-dihydroxyindole, 4-hydroxy-5-methylindole, 2-carboxyl-6-hydroxyindole, 6-hydroxy-N-methylindole, 2-ethoxycarbonyl-5,6-dihydroxyindole, 4-hydroxy-7-methoxy-2,3-dimethylindole, 4-hydroxy-5-ethoxy-N-methylindole, 6-hydroxy-5-methoxy-2-methylindole, 6-hydroxy-5-methoxy-2,3-dimethylindole, 6-hydroxy-2-ethoxycarbonylindoie, 7-hydroxy-3-methylindole, 5-hydroxy-6-methoxy-2,3-dimethylindole, 5-hydroxy-3-methylindole 5-acetoxy-6-hydroxyindole, 5-hydroxy-2-ethoxycarbonylindole, 6-hydroxy-2-carboxyl-5-methylindole, 6-hydroxy-2-ethoxycarbonyl-5-methoxyindole, 6-N-β-hydroxyethylaminoindole, 4-aminoindole, 5-aminoindole, 6-aminoindole 7-aminoindole, N-methyl-6-hydroxyethylaminoindole, 6-amino-2,3-dimethylindole, 6-amino-2,3,4,5-tetramethylindole, 6-amino-2,3,4-trimethylindole, 6-amino-2,3,5-trimethylindole, 6-amino-2,3,6-trimethylindole, 5,6-diacetoxyindole, 5-methoxy-6-acetoxyindole and 5,6-dimethoxyindole, and the addition salts of these compounds.

**20.** Method according to Claim 17, characterized in that there is chosen, as precursor, at least one indoline compound of formula:

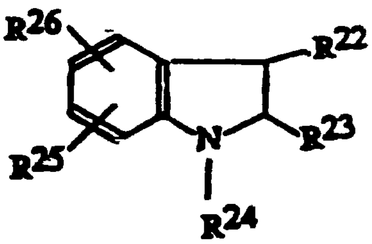

(VII)

in which formula:

- $R^{22}$ and $R^{24}$ represent, independently of each other, a hydrogen atom or a $C_1$-$C_4$ alkyl radical;
- $R^{23}$ represents a hydrogen atom, a $C_1$-$C_4$ alkyl radical, a carboxyl group or a ($C_1$-$C_4$ alkoxy)carbonyl group;
- $R^{25}$ denotes a hydrogen atom, a $C_1$-$C_4$ alkyl radical, a hydroxyl group, a ($C_1$-$C_4$)alkoxy group, an amino group or a $C_1$-$C_{10}$ alkylamino group or a halogen atom;
- $R^{26}$ denotes a hydrogen atom, a hydroxyl group, a $C_1$-$C_4$ alkoxy group or an amino group; on the condition that at least one of the radicals $R^{25}$ or $R^{26}$ denotes a hydroxyl, alkoxy or amino group; provided that when $R^{26}$ denotes an amino group, $R^{25}$ cannot denote an alkylamino radical;
- it being possible for $R^{25}$ and $R^{26}$ to also form a $C_1$-$C_2$ alkylenedioxy ring when they are at the 5 and 6 positions; and/or at least one of their alkali metal, alkaline-earth metal, ammonium or amine salts and their hydrochlorides hydrobromides, sulphates and methanesulphonates.

**21.** Method according to Claim 20, characterized in that the indoline compound is chosen from the group consisting of 5,6-dihydroxyindoline, 6-hydroxyindoline, 5,6-methylenedioxyindoline, 7-methoxy-6-hydroxyindoline, 6,7-dihydroxyindoline, 5-hydroxy-4-methoxyindoline, 4,5-dihydroxyindoline, 5-methoxy-6-hydroxyindoline, 4-hydroxy-5-methoxyindoline, 5-hydroxy-6-methoxyindoline, 4,7-dihydroxyindoline, 6-aminoindoline, N-ethyl-4-hydroxyindoline, 1-ethyl-6-aminoindoline, 5,6-diaminoindoline, 1-methyl-6-aminoindoline, 2-methyl-6-aminoindoline, 3-methyl-6-aminoindoline, 2-methyl-5,6-diaminoindoline, 5-chloro-7-aminoindoline, 3-methyl-5,7-diaminoindoline, 5,7-diaminoindoline, 2-methyl-5,7-diaminoindoline, 7-aminoindoline 2-methyl-7-aminoindoline, 4-aminoindoline, 4-amino-6-chloroindoline, 4-amino-6-iodoindoline, 4-amino-5-bromoindoline, 4-amino-5-hydroxyindoline, 4-amino-7-hydroxyindoline, 4-amino-5-methoxyindoline, 4-amino-7-methoxyindoline, 5-aminoindoline, 2,3-dimethyl-5-aminoindoline, 1-methyl-5-aminoindoline, 2-methyl-5-aminoindoline, 5-N-(1-methylhexyl)aminoindoline, 5,6-dimethoxyindoline and 5,6-dihydroxy-2-carboxyindoline.

**22.** Method according to Claim 16, characterized in that particles of polymers of acrylamide and bis-acrylamide are prepared.

**23.** Method according to one of Claims 1 to 16, characterized in that the submicron particles are metal oxides.

**24.** Method according to Claim 23, characterized in that the submicron particles are silver, iron or zinc oxides.

**25.** Method according to one of Claims 1 to 16, characterized in that the submicron particles are metal sulphides.

**26.** Method according to Claim 25, characterized in that the submicron particles are cadmium, zinc, lead or copper sulphides.

27. Method according to one of Claims 1 to 22, characterized in that to prepare the lamellar phase, the lipid(s) which should form the wall of the vesicles are solubilized in a solvent, the solvent is evaporated under reduced pressure and then the lipid association thus formed is mixed with the aqueous phase E, the mixture is homogenized, heated at a temperature of between 10 and 150°C for a period of between 0.25 and 2 h and allowed to return to room temperature the cycle: homogenization-heating-return to room temperature being repeated at least once.

28. Method according to Claim 27, characterized in that dichloromethane, chloroform, ethyl acetate, butyl acetate, ethyl formate, hexane, cyclohexane, toluene, petroleum ether, methanol, ethanol, propanol, methyl ether, ethyl ether and mixtures of at least two of them, are used as solvent.

29. Composition obtained by the method according to one of Claims 1 to 28.

30. Composition according to Claim 29, comprising an aqueous medium where lipid vesicles and submicron particles are dispersed, the said particles being contained in the said vesicles and/or independent thereof, characterized in that the volume of the said vesicles and, where appropriate, of the particles independent thereof, represents 5 to 90% of the medium, the vesicular lipids representing from 1 to 90% by volume relative to the total volume of the composition, the vesicles having sizes of between 20 and 3000 nm

31. Composition according to either of Claims 29 and 30, characterized in that it contains, in an aqueous medium, from 0.1 to 20% by weight of particles of pigments having sizes of between 20 and 500 nm.

## Patentansprüche

1. Verfahren zur Herstellung von Submikronteilchen in Gegenwart von Lipidvesikeln, ausgehend von wenigstens einem Präkursor der Teilchen, ausgehend von wenigstens einem Lipid und einer ersten wäßrigen Phase, von der ein Teil dazu bestimmt ist, in den Vesikeln eingekapselt zu werden, und gegebenenfalls einer zweiten wäßrigen Phase, die dazu bestimmt ist, Teil des Dispersionsmediums der Vesikel zu sein, dadurch gekennzeichnet, daß man vor und/oder während und/oder nach der Herstellung der Vesikelphase wenigstens einen Präkursor der Teilchen in die erste wäßrige Phase und/oder die zweite wäßrige Phase gibt, wobei wenigstens ein Agens, das in der Lage ist, den (oder die) Präkursor(en) in die gewünschten Teilchen umzuwandeln, auf diesen (diese) nach dessen (deren) Einführung in das Herstellungsmedium einwirkt, wobei der in der wäßrigen Dispersionsphase enthaltene Präkursor nicht vor Einführung des Agens eliminiert wird, so daß die wäßrige Dispersionsphase präzipitierte Submikronteilchen enthält.

2. Verfahren nach Anspruch 1, dadurch gekennzeichnet, daß man die Homogenisation und das Bewegen so durchführt, daß man Vesikel mit einer Größe im Bereich von 20 bis 3000 nm erhält.

3. Verfahren nach einem der Ansprüche 1 oder 2, dadurch gekennzeichnet, daß man eine lamellare Phase herstellt, worin die Konzentration an Lipid(en) im Bereich von 10 bis 90 Gew.-% liegt.

4. Verfahren nach einem der Ansprüche 1 bis 3, dadurch gekennzeichnet, daß man eine Vesikelphase herstellt, worin die Konzentration an Lipiden im Bereich von 1 bis 90 Gew.-% liegt.

5. Verfahren nach einem der Ansprüche 1 bis 4, dadurch gekennzeichnet, daß man die Vesikelphase bei einer Temperatur im Bereich von 10 bis 150°C herstellt.

6. Verfahren nach einem der Ansprüche 1 bis 5, dadurch gekennzeichnet, daß man wenigstens einen Präkursor verwendet, der in der Lage ist, unter Einwirkung von wenigstens einem Fällungsmittel als Submikronteilchen auszufallen.

7. Verfahren nach einem der Ansprüche 1 bis 5, dadurch gekennzeichnet, daß man als Präkursor wenigstens ein polymerisierbares Monomer verwendet, auf welches man wenigstens ein Polymerisationsmittel einwirken läßt.

8. Verfahren nach einem der Ansprüche 1 bis 7, dadurch gekennzeichnet, daß man die Umwandlung des Präkursors (oder der Präkursoren) nach Bildung der Vesikel durchführt.

**9.** Verfahren nach einem der Ansprüche 1 bis 8, dadurch gekennzeichnet, daß das Lipid (die Lipide) ausgewählt ist (sind) unter amphiphilen, nicht-ionischen Lipiden, amphiphilen, ionischen Lipiden und Gemischen von nicht-ionischen und ionischen Lipiden.

**10.** Verfahren nach Anspruch 9, dadurch gekennzeichnet, daß die amphiphilen, nicht-ionischen Lipide ausgewählt sind unter:

(1) geradkettigen oder verzweigten Polyglycerinderivaten der Formel:

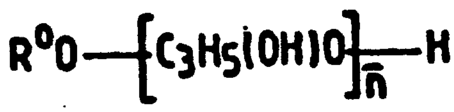 (I)

worin

. $-C_3H_5(OH)O$ durch die folgenden Strukturen, im Gemisch oder einzeln, repräsentiert wird:

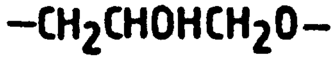 , 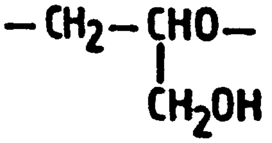 , 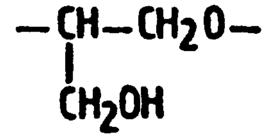

. $\bar{n}$ für einen statistischen Mittelwert im Bereich von 1 bis 6 steht;

. $R^0$ bedeutet:

(a) eine aliphatische, geradkettige oder verzweigte, gesättigte oder ungesättigte Kette mit 12 bis 30 Kohlenstoffatomen; oder Kohlenwasserstoffreste von Lanolinalkoholen; oder die Reste von langkettigen alpha-Diolen;

(b) einen Rest $R^1CO$, worin $R^1$ für einen geradkettigen oder verzweigten, aliphatischen $C_{11}$-$C_{29}$-Rest steht;

(c) einen Rest $R^2[OC_2H_3(R^3)]$ oder

. $R^2$ die für $R^0$ angegebene Bedeutung (a) oder (b) besitzen kann;

. $OC_2H_3(R^3)$- durch eine der folgenden Strukturen, im Gemisch oder einzeln, repräsentiert wird:

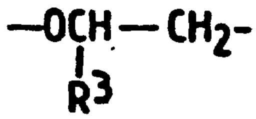 und 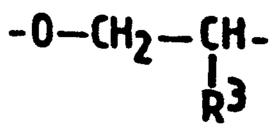

worin $R^3$ die für $R^0$ angegebene Bedeutung (a) besitzt;

(2) geradkettigen oder verzweigten Polyglycerinethern, die zwei Fettketten aufweisen;
(3) polyoxyethylenierten Fettalkoholen und polyoxyethylenierten Sterolen und Phytosterolen;
(4) Polyolethern;
(5) gegebenenfalls oxyethylenierten Polyolestern;
(6) Glycolipiden natürlicher oder synthetischer Herkunft, insbesondere Glucoseethern und -estern;
(7) Hydroxyamiden der Formel (II)

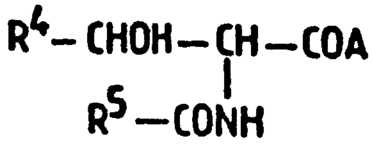

(II)

worin

- $R^4$ für einen $C_7$-$C_{21}$-Alkyl- oder -Alkenylrest steht;
- $R^5$ für einen gesättigten oder ungesättigten $C_7$-$C_{31}$-Kohlenwasserstoffrest steht;
- COA für eine Gruppe steht, die unter folgenden zwei Gruppen ausgewählt ist:

  . einem Rest der Formel

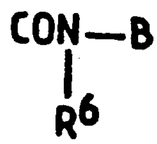

  worin
  . B für einen von primären oder sekundären, mono- oder polyhydroxylierten Aminen abgeleiteten Alkylrest steht; und
  . $R^6$ für ein Wasserstoffatom oder einen Methyl-, Ethyl- oder Hydroxyethylrest steht; und
  . einem Rest -COOZ, worin Z für einen $C_3$-$C_7$-Polyolrest steht.

(8) Glycerinderivaten der Formel

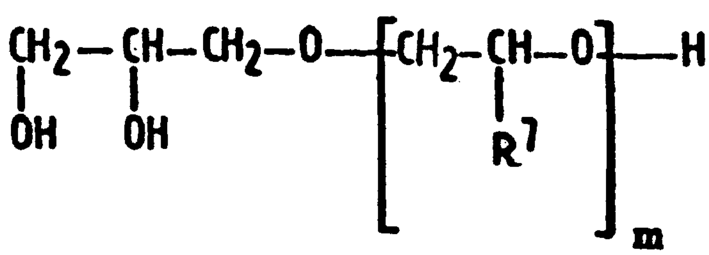

(III)

worin $R^7$ für einen geradkettigen $C_{14}$-$C_{18}$-Alkylrest oder eine Gruppe $CH_2A$ steht, worin A für $-OR_{14}$ und $R_{14}$ für einen geradkettigen $C_{10}$-$C_{18}$-, vorzugsweise einen $C_{16}$-Alkylrest, steht und m ein statistischer Mittelwert größer 1 und höchstens gleich 3 ist und m zusätzlich für einen reellen (nicht-statistischen) Wert von 2 stehen kann, wenn $R_7 = -CH_2A$ ist;
(9) Glukoseestern der Formel

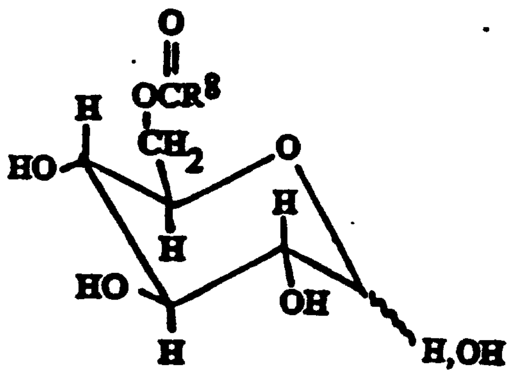

(IV)

worin $R^8$ für eine geradkettige, gesättigte oder ungesättigte Kohlenwasserstoffkette mit 9 bis 17 Kohlenstoffatomen steht.

11. Verfahren nach Anspruch 9, dadurch gekennzeichnet, daß die amphiphilen ionischen Lipide ausgewählt sind unter natürlichen Phospholipiden, synthetischen Phospholipiden, anionischen Verbindungen, kationischen quaternären Ammonium-Derivaten der Formel

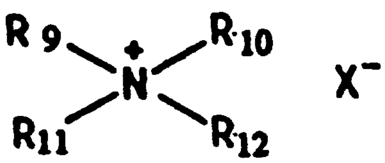

(V)

worin $R_9$ und $R_{10}$, die gleich oder verschieden sein können, für $C_{12}$-$C_{20}$-Alkylreste stehen und $R_{11}$ und $R_{12}$, die gleich oder verschieden sein können, für $C_1$-$C_4$-Alkylreste stehen; polymerisierbaren Lipiden.

12. Verfahren nach einem der Ansprüche 9 bis 11, dadurch gekennzeichnet, daß man den Lipiden Additive zusetzt, die ausgewählt sind unter

- langkettigen Alkoholen und Diolen,
- Sterolen,
- langkettigen Aminen und deren quaternären Ammoniumderivaten,
- Dihydroxyalkylaminen, polyoxyethylenierten Fettaminen, langkettigen Aminoalkoholestern und deren Salzen und quaternären Ammoniumderivaten,
- Phosphorsäureestern von Fettalkoholen; Alkylsulfaten und
- Polypeptiden und Proteinen.

13. Verfahren nach einem der Ansprüche 9 bis 12, dadurch gekennzeichnet, daß man der entsprechenden Lipidphase der Vesikel wenigstens ein Lipoprotid zusetzt, das keine Sulfhydrylgruppe aufweist und ausgewählt ist unter mono- oder polyacylierten Aminosäure- oder Polypeptidderivaten, deren Acylrest $R_{13}$-CO eine $C_{13}$-$C_{19}$-Kohlenwasserstoffkette $R_{13}$ aufweist, wobei wenigstens eine der Gruppen, die die Polypeptidkette oder den Aminosäurerest mit der lipophilen Kette verbindet, eine Amidfunktion ist und die Carboxylgruppen der Polypeptidkette oder des Aminosäurerestes teilweise oder völlig durch ein oder mehrere Alkalimetall-Kationen, ein Ammoniumion oder substituiertes, von einem Amin abgeleitetes Ammoniumion neutralisiert sein kann, wobei das Lipoprotid (die Lipoprotide) in einer Menge von 1 bis 15 Gew.-%, bezogen auf das Gesamtgewicht der entsprechenden Lipidphase, und/oder wenigstens ein Cholesterinsulfat mit einem Ammonium-, Alkali- oder Erdalkalikation in einer Menge von 1 bis 50 Gew.-%, bezogen auf das Gewicht der Lipidphase an sich und/oder wenigstens ein Cholesterinphosphat in Form der freien Säure oder neutralisiert durch ein Ammonium-, Alkali- oder Erdalkalikation in einer Menge von 1 bis 40 Gew.-%, bezogen auf das Gesamtgewicht der Lipidphase an sich, vorhanden ist (sind).

14. Verfahren nach einem der Ansprüche 1 bis 13, dadurch gekennzeichnet, daß man als wäßrige Phase E oder D Wasser oder ein Gemisch aus Wasser und wenigstens einem mit Wasser mischbaren Lösungsmittel verwendet.

**15.** Verfahren nach Anspruch 14, dadurch gekennzeichnet, daß man das (oder die) mit Wasser mischbare(n) Lösungsmittel unter $C_1$-$C_7$-Alkoholen und $C_1$-$C_5$-Alkylpolyolen auswählt.

**16.** Verfahren nach einem der Ansprüche 1 bis 15, wobei man Submikronteilchen herstellt, die aus Pigmenten oder Polymeren gebildet sind, dadurch gekennzeichnet, daß man Fällungs- oder Polymerisationsmittel verwendet, die auf chemischer und physikalisch-chemischer Ebene mit den Vesikeln kompatibel und unter Oxidantien, freiradikalischen Initiatoren, Enzymen, Strahlen, Säuren und Basen ausgewählt sind.

**17.** Verfahren nach Anspruch 16, wobei man Pigmentteilchen in Submikrongröße herstellt, dadurch gekennzeichnet, daß man als Präkursor wenigstens eine Indolverbindung und/oder wenigstens eine Indolinverbindung wählt, die man einer Oxidation oder einer oxidativen oder enzymatischen Polymerisation unterwirft.

**18.** Verfahren nach Anspruch 17, dadurch gekennzeichnet, daß man als Präkursor wenigstens eine Indolverbindung der Formel

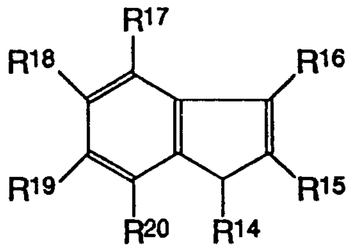

(VI)

worin

- $R^{14}$ und $R^{16}$ unabhängig voneinander für Wasserstoff oder einen $C_1$-$C_4$-Rest stehen;
- $R^{15}$ für Wasserstoff, einen $C_1$-$C_4$-Alkylrest, eine Carboxylgruppe oder ($C_1$-$C_4$-Alkoxy)-Carbonylgruppe steht;
- $R^{17}$ und $R^{20}$ unabhängig voneinander für Wasserstoff, eine Hydroxygruppe, eine $C_1$-$C_4$-Alkylgruppe, eine Amino-, $C_1$-$C_4$-Alkoxy-, ($C_1$-$C_4$-Alkoxy)oxy- oder ($C_2$-$C_4$-Acyl)aminogruppe stehen;
- $R^{18}$ für Wasserstoff, eine Hydroxygruppe, $C_1$-$C_4$-Alkoxy, einen $C_1$-$C_4$-Alkylrest, eine Halogen-, Amino-, ($C_2$-$C_4$-Acyl)oxy-, ($C_2$-$C_4$-Acyl)amino- oder Trimethylsilyloxygruppe steht;
- $R^{19}$ für Wasserstoff, eine Hydroxygruppe, $C_1$-$C_4$-Alkoxy, Amino, ($C_2$-$C_4$-Acyl)oxy-, ($C_2$-$C_4$-Acyl)amino, Trimethylsilyloxy oder ($C_2$-$C_4$-Hydroxyalkyl)amino steht;
- wobei $R^{18}$ und $R^{19}$ zusammen mit den Kohlenstoffatomen, an die sie gebunden sind, auch einen gegebenenfalls mit einer $C_1$-$C_4$-Alkylgruppe oder einer $C_1$-$C_4$-Alkoxygruppe substituierten Methylendioxyring oder einen Carbonyldioxyring bilden können;

mit der Maßgabe, daß wenigstens einer der Reste $R^{17}$ bis $R^{20}$ für eine Gruppe OZ oder $NHR^{21}$ steht, wobei höchstens einer der Reste $R^{17}$ bis $R^{20}$ für $NHR^{21}$ steht; und höchstens zwei der Reste $R^{17}$ bis $R^{20}$ für OZ stehen, und sich die beiden OH-Gruppen, wenn Z für Wasserstoff steht, in 5- und 6-Stellung befinden; und wenigstens einer der Reste $R^{17}$ bis $R^{20}$ für Wasserstoff steht, und wenn nur einer dieser Reste für Wasserstoff steht, dann steht nur einer der Reste $R^{17}$ bis $R^{20}$ für $NHR^{21}$ oder OZ und die anderen Reste stehen für einen $C_1$-$C_4$-Alkylrest; wobei $R^{21}$ für Wasserstoff, eine $C_2$-$C_4$-Acylgruppe, eine $C_2$-$C_4$-Hydroxyalkylgruppe und Z für Wasserstoff, eine $C_2$-$C_{14}$-Acylgruppe, eine $C_1$-$C_4$-Alkylgruppe oder eine Trimethylsilylgruppe steht;

und/oder eines der Alkalimetall-, Erdalkalimetall-, Ammonium- oder Amin-, Chlorwasserstoff-, Bromwasserstoff-, Sulfat- oder Methansulfonatsalze davon auswählt.

**19.** Verfahren nach Anspruch 18, dadurch gekennzeichnet, daß die Indolverbindung ausgewählt ist unter 4-Hydroxyindol, 5-Hydroxyindol, 6-Hydroxyindol, 7-Hydroxyindol, 4-Hydroxy-5-methoxyindol, 4-Hydroxy-5-ethoxyindol, 2-Carboxy-5-hydroxyindol, 5-Hydroxy-6-methoxyindol, 6-Hydroxy-7-methoxyindol, 5-Methoxy-6-hydroxyindol, 5,6-Dihydroxyindol, N-Methyl-5,6-dihydroxyindol, 2-Methyl-5,6-hydroxyindol, 3-Methyl-5,6-hydroxyindol, 2,3-Dimethyl-5,6-dihydroxyindol, 2-Carboxyl-5,6-dihydroxyindol, 4-Hydroxy-5-methylindol, 2-Carboxyl-6-hydroxyindol, 6-Hydroxy-N-methylindol, 2-Ethoxycarbonyl-5,6-dihydroxyindol, 4-Hydroxy-7-methoxy-2,3-dimethylindol, 4-Hydroxy-5-ethoxy-N-methylindol, 6-Hydroxy-5-methoxy-2-methylindol, 6-Hydroxy-5-methoxy-2,3-dimethylindol, 6-Hydroxy-2-ethoxycarbonylindol, 7-Hydroxy-3-methylindol, 5-Hydroxy-6-methoxy-2,3-dimethylindol, 5-Hydroxy-3-methylindol, 5-Acetoxy-6-hydroxyindol, 5-Hydroxy-2-ethoxycarbonylindol, 6-Hydroxy-2-carboxyl-5-methylindol, 6-Hydroxy-2-ethoxycarbonyl-5-methoxyindol, 6-N-β-Hydroxyethylaminoindol, 4-Aminoindol, 5-Aminoindol, 6-Aminoindol, 7-Aminoindol, N-Methyl-6-Hydroxyethylaminoindol, 6-Amino-2,3-dimethylindol, 6-Amino-2,3,4,5-tetramethylindol, 6-Amino-2,3,4-trimethylindol, 6-Amino-2,3,5-trimethylindol, 6-Amino-2,3,6-trimethylindol, 5,6-Diacetoxyindol, 5-Methoxy-6-acetoxyindol und 5,6-Dimethoxyindol und den Additionssalzen dieser Verbindungen.

**20.** Verfahren nach Anspruch 17, dadurch gekennzeichnet, daß man als Präkursor wenigstens eine Indolinverbindung der Formel

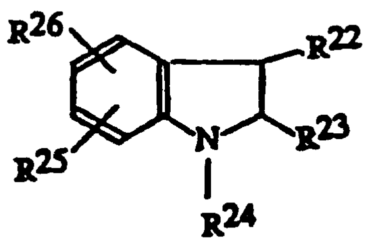

(VII)

worin

- $R^{22}$ und $R^{24}$ unabhängig voneinander für ein Wasserstoffatom oder einen $C_1$-$C_4$-Alkylrest stehen;
- $R^{23}$ für ein Wasserstoffatom, einen $C_1$-$C_4$-Alkylrest, eine Carboxyl- oder ($C_1$-$C_4$)-Alkoxycarbonylgruppe steht;
- $R^{25}$ für ein Wasserstoffatom, einen $C_1$-$C_4$-Alkylrest, Hydroxyl, ($C_1$-$C_4$)Alkoxy, $C_1$-$C_{10}$-Amino oder -Alkylamino oder Halogen steht;
- $R^{26}$ für ein Wasserstoffatom, eine Hydroxylgruppe, $C_1$-$C_4$-Alkoxy oder Amino steht; mit der Maßgabe, daß wenigstens einer der Reste $R^{25}$ und $R^{26}$ für eine Hydroxyl-, Alkoxy- oder Aminogruppe steht; und mit der Maßgabe, daß $R^{25}$ nicht für einen Alkylaminorest stehen kann, wenn $R^{26}$ für eine Aminogruppe steht;
- wobei $R^{25}$ und $R^{26}$, wenn sie sich in 5- und 6-Stellung befinden, auch einen $C_1$-$C_2$-Alkylendioxyring bilden können; und/oder wenigstens eines der Alkalimetall-, Erdalkalimetall-, Ammonium- oder Amin-, Chlorwasserstoff-, Bromwasserstoff-, Sulfat- oder Methansulfonatsalze davon wählt.

**21.** Verfahren nach Anspruch 20, dadurch gekennzeichnet, daß die Indolinverbindung ausgewählt ist unter 5,6-Dihydroxyindolin, 6-Hydroxyindolin, 5,6-Methylendioxyindolin, 7-Methoxy-6-hydroxyindolin, 6,7-Dihydroxyindolin, 5-Hydroxy-4-methoxyindolin, 4,5-Dihydroxyindolin, 5-Methoxy-6-hydroxyindolin, 4-Hydroxy-5-methoxyindolin, 5-Hydroxy-6-methoxyindolin, 4,7-Dihydroxyindolin, 6-Aminoindolin, N-Ethyl-4-hydroxyindolin, 1-Ethyl-6-aminoindolin, 5,6-Diaminoindolin, 1-Methyl-6-aminoindolin, 2-Methyl-6-aminoindolin, 3-Methyl-6-aminoindolin, 2-Methyl-5,6-diaminoindolin, 5-Chlor-7-aminoindolin, 3-Methyl-5,7-diaminoindolin, 5,7-Diaminoindolin, 2-Methyl-5,7-diaminoindolin, 7-Aminoindolin, 2-Methyl-7-aminoindolin, 4-Aminoindolin, 4-Amino-6-chlorindolin, 4-Amino-6-iodindolin, 4-Amino-5-bromindolin, 4-Amino-5-hydroxyindolin, 4-Amino-7-hydroxyindolin, 4-Amino-5-methoxyindolin, 4-Amino-7-methoxyindolin, 5-Aminoindolin, 2,3-Dimethyl-5-aminoindolin, 1-Methyl-5-aminoindolin, 2-Methyl-5-aminoindolin, 5-N-(1-Methylhexyl)aminoindolin, 5,6-Dimethoxyindolin und 5,6-Dihydroxy-2-carboxyindolin.

**22.** Verfahren nach Anspruch 16, dadurch gekennzeichnet, daß man Teilchen aus Acrylamid- und Bisacrylamid-Polymeren herstellt.

**23.** Verfahren nach einem der Ansprüche 1 bis 16, dadurch gekennzeichnet, daß die Submikronteilchen Metalloxide sind.

**24.** Verfahren nach Anspruch 23, dadurch gekennzeichnet, daß die Submikronteilchen Silber-, Eisen- oder Zinkoxide sind.

**25.** Verfahren nach einem der Ansprüche 1 bis 16, dadurch gekennzeichnet, daß die Submikronteilchen Metallsulfide sind.

**26.** Verfahren nach Anspruch 25, dadurch gekennzeichnet, daß die Submikronteilchen Cadmium-, Zink-, Blei- oder Kupfersulfide sind.

**27.** Verfahren nach einem der Ansprüche 1 bis 22, dadurch gekennzeichnet, daß man zur Herstellung der lamellaren Phase das (die) Lipid(e) vor der Bildung der Vesikelwand in einem Lösungsmittel solubilisiert, das Lösungsmittel unter reduziertem Druck verdampft, dann die so gebildete Lipidassoziation mit der wäßrigen Phase E vermischt, homogenisiert, über einen Zeitraum im Bereich von 0,25 bis 2 Stunden auf eine Temperatur im Bereich von 10 bis 150°C erwärmt und dann auf Umgebungstemperatur abkühlen läßt, wobei man den Zyklus von Homogenisation - Erwärmen - Abkühlen auf Umgebungstemperatur wenigstens einmal wiederholt.

**28.** Verfahren nach Anspruch 27, dadurch gekennzeichnet, daß man als Lösungsmittel Dichlormethan, Chloroform, Ethylacetat, Butylacetat, Ethylformiat, Hexan, Cyclohexan, Toluol, Petrolether, Methanol, Ethanol, Propanol, Methylether, Ethylether und Gemische von wenigstens zwei dieser Verbindungen verwendet.

**29.** Zusammensetzung, erhältlich durch ein Verfahren nach einem der Ansprüche 1 bis 28.

**30.** Zusammensetzung nach Anspruch 29, umfassend ein wäßriges Medium, worin Vesikellipide und Submikronteilchen dispergiert sind, wobei die Teilchen in den Vesikeln enthalten und/oder von diesen unabhängig sind, dadurch gekennzeichnet, daß das Volumen der Vesikel, und gegebenenfalls der von diesen unabhängigen Teilchen, 5 bis 90 % des Mediums ausmacht, wobei die Vesikellipide 1 bis 90 Volumen-%, bezogen auf das Gesamtvolumen der Zusammensetzung, ausmachen und die Vesikel eine Größe im Bereich von 20 bis 3000 nm besitzen.

**31.** Zusammensetzung nach einem der Ansprüche 29 oder 30, dadurch gekennzeichnet, daß sie 0,1 bis 20 Gew.-% Pigmentteilchen mit einer Größe im Bereich von 20 bis 500 nm in wäßrigem Medium enthält.